# EUROPÄISCHE PATENTSCHRIFT

(11) **EP 0 698 665 B1**
(45) Veröffentlichungstag und Bekanntmachung des Hinweises auf die Patenterteilung: **20.05.1998**
(21) Anmeldenummer: 95111698.7
(22) Anmeldetag: 25.07.1995
(51) Int. Cl.: C12N 15/11, C12N 15/33, C12N 15/63, C12N 5/10, A61K 48/00

(54) **Infektiöser cDNA-Klon des Tick-Borne Enzephalitis (TBE)-Virus, davon abgeleiteter rekombinanter Impfstoff und Herstellung desselben**
Infectious Tick-Borne Encephalitis virus cDNA clone, recombinant vaccine derived therefrom and preparation thereof
Clone ADNc du virus de l'encephalite de Tick-Borne, vaccins recombinant dérivés de celui-ci et production de ceux-ci

(30) Priorität: 27.07.1994 DE 4426622
(43) Veröffentlichungstag der Anmeldung: 28.02.1996
(73) Patentinhaber: IMMUNO AG, 1221 Wien (AT)
(72) Erfinder: Heinz, Franz Xaver, Prof.Dr., A-1080 Wien (AT); Kunz, Christian, Prof.Dr., A-1180 Wien (AT); Mandl, Christian, Doz.Dr., A-1160 Wien (AT)
(74) Vertreter: Kolb, Helga, Dr. Dipl.-Chem.

(56) Entgegenhaltungen:
- VIROLOGY, Bd. 173, 1989, Seiten 291-301, XP002007883 C.W. MANDL ET AL.: "Genome sequence of Tick-borne encephalitis virus (western subtype) and comparative analysis of nonstrucutral proteins with other flaviviruses"
- MOLEKULARNAYA GENETIKA, MIKROBIOLOGIYA I. VIRUSOLOGIYA (USSR), Bd. 4, 1991, Seiten 23-29, XP002007884 P.F. SAFRONOV ET AL.: "Nucleotide sequence of genes and complete aminoacid sequence of proteins of tick-borne encephalitis virus strain 205"
- JOURNAL OF VIROLOGY, Bd. 65, Nr. 8, 1991, Seiten 4070-4077, XP002007885 C.W. MANDL ET AL.: "Presence of poly(a) in a flavivirus: significant differences between the 3' non-coding regions of the genomic RNAs of tick-borne encephalitis virus strains"

## Beschreibung

Die vorliegende Erfindung betrifft ein Verfahren zur rekombinanten Herstellung von Impfstoff gegen Tick-Borne Enzephalitis (TBE)-Virus, ein Verfahren zur Herstellung desselben sowie die dieser Herstellung zugrundeliegenden Nukleinsäuren und transformierten Wirtszellen. Insbesondere betrifft die Erfindung eine komplette cDNA, die für ein infektiöses TBEV-RNA-Transkript kodiert. Mit Hilfe des erfindungsgemässen Verfahrens ist es möglich attenuierte TBE-Viren herzustellen, die sich in besonderem Masse zur Impfstoffproduktion eignen.

Das Tick-Borne Enzphalitis (TBE)-Virus ist ein Vertreter der Familie Flaviviridae. Diese Virusfamilie gliedert sich in drei Genera, nämlich das Genus Pestiviren, das Genus Hepatitis C-Viren und das Genus Flaviviren. Der Begriff 'Flaviviren' wird hier stets synonym für das Genus, nicht die Familie, verwendet.

Die Flaviviren umfassen ungefähr 70 bekannte Vertreter, von denen einige die Erreger bedeutender Erkrankungen des Menschen sind. Dazu zählen insbesondere das Gelbfieber-Virus, die Dengue-Viren (Typ 1-4), das Japan B-Virus und das TBE-Virus. Während die erstgenannten Viren sowie die Mehrzahl der übrigen Flaviviren durch Stechmücken auf den Menschen bzw. verschiedene Wirbeltiere übertragen werden, bilden die 'Tick-Borne', also durch Zecken übertragenen TBE-Viren eine biologisch-ökologisch und epidemiologisch distinkte Untergruppe innerhalb der Flaviviren. Die TBE-Viren unterscheiden sich auch in bestimmten molekularbiologischen, genetischen Eigenschaften von den anderen Flaviviren.

Das Genom der Flaviviren ist ein einzelsträngiges, ungefähr 11.000 Nukleotide langes RNA-Molekül. Dieses Molekül ist positiv-strängig, d.h. es entspricht in seiner Orientierung einer mRNA. Von diesem RNA-Molekül werden alle viralen Proteine an den Ribosomen abgelesen (translatiert). Das RNA-Genom der Flaviviren ist infektiös, d.h. wird virale RNA ohne irgendwelche andere Komponenten des Viruspartikels mit geeigneten Methoden in infizierbare Zellen eingebracht, so führt dies zu einer produktiven Virusinfektion, also der Bildung neuer, infektiöser Viruspartikel.

Gegen eine Reihe von durch Flaviviren verursachte Erkrankungen befinden sich Impfstoffe in Verwendung oder Entwicklung. Prinzipiell können zwei verschiedene Arten von Impfstoffen unterschieden werden, nämlich Lebend- und Totimpfstoffe.

Lebendimpfstoffe sind in ihrer Virulenz verminderte (= attenuierte) Virusstämme, die kein oder nur ein sehr schwaches Krankheitsbild hervorrufen (apathogen sind), aber eine auch gegen das virulente Wildvirus schützende Immunantwort bewirken. Bei den meisten bis heute verwendeten Lebenimpfstoffen handelt es sich entweder um in der Natur vorkommende Viren (z.B. Vacciniavirus) oder um Virusstämme, die im Labor durch wiederholtes Passagieren in verschiedenen biologischen Systemen verändert wurden (z.B. Polio-, Gelbfieberviren). Bei Flaviviren ist ein Lebendimpfstoff gegen des Gelbfieber-Virus seit den 30er Jahren in Verwendung. Lebendimpfstoffe gegen die vier Dengue Virustypen befinden sich in klinischer Erprobung.

Bei Totimpfstoffen handelt es sich um ursprünglich virulente Viren, die in geeigneten biologischen Systemen gezüchtet und dann durch ein chemisches Verfahren (z.B. Behandlung mit Formaldehyd) inaktiviert werden. Totimpfstoffe erfordern bei ihrer Produktion das Arbeiten mit grossen Mengen von virulenten, pathogenen Viren. Durch die Inaktivierung können Struktureigenschaften des Viruspartikels verändert werden. Dies kann zu einer weniger spezifischen und weniger effizienten Immunantwort führen. Bei der Verwendung von Totimpfstoffen kommt es zu keiner Infektion des Geimpften, es wird aber eine Immunantwort induziert, die auch gegen replikationskompetentes, virulentes Virus schützt. Solche Totimpfstoffe sind gegen das TBE-Virus und gegen das Japan B-Virus in Verwendung (Monath, T. 1990).

Im Hinblick auf die Impfstoffherstellung ist bei RNA-Viren deren hohe Mutationsfrequenz zu berücksichtigen. Aufgrund dieser hohen Mutationsfrequenz ist ein zur Produktion eines Impfstoffes verwendetes Saatvirus immer heterogen und kann sich durch Passagierung ebenfalls ständig verändern. Dadurch kann es zu Veränderungen der Eigenschaften des Virus kommen, wodurch sich produktionstechnische und sicherheitstechnische Probleme ergeben können. Verschiedene in Verwendung befindliche Saatviren des Gelbfieber 17D-Impfstoffes weisen bereits erhebliche Sequenzunterschiede auf, die auch biologische Unterschiede vermuten lassen (Post et al, 1992; Ledger et al, 1992).

Aufgrund des Vorhergesagten ergibt sich ohne weiteres, dass es vorteilhaft ist, bei der Impfstoffherstellung anstelle eines heterogenen Saatvirus von einem einheitlichen cDNA-Klon auszugehen. Darüber hinaus ist es von Vorteil, auch für die Herstellung eines Totimpfstoffes einen spezifischer attenuierten Virusstamm einzusetzen.

Ohne einen infektiösen cDNA-Klon können bei RNA-Viren attenuierte Virusstämme nur entweder zufällig aus der Natur isoliert werden, oder sie werden durch Selektion zufälliger Mutanten durch wiederholte Passagierung im Labor gezüchtet. Auf Basis dieser Virusstämme kann man das molekulare Prinzip der Attenuierung, ihr Ausmass und die Wahrscheinlichkeit einer Reversion zum virulenten Wildtyp (letzteres stellt den wichtigsten Sicherheitsfaktor eines Lebendimpfstoffes dar) praktisch nicht beeinflussen. Als Folge davon gibt es derzeit keinen Stamm des TBE-Virus, der als Lebendimpfstoff geeignet ist.

Im Hinblick auf eine gentechnologische Veränderung und Gewinnung verschiedener Flaviviren ist bekannt, dass es schwierig bis unmöglich ist, ein cDNA-Molekül mit der gesamten Information eines Flavivirus-Genoms stabil z.B. in E. coli zu klonieren. Bei diesen Arbeiten wurde ausserdem beobachtet, dass spontan Mutationen auftreten, die dazu führen, dass keine infektiöse RNA gewonnen werden kann. Man nimmt an, dass bestimmte Sequenzelemente für die Bakterien, in denen die cDNA-Klone vermehrt werden sollen, toxisch sind. Hierbei sind die auftretenden Probleme jedoch sehr unterschiedlich: Während für Dengue-4 (Lai et al, 1991) und Japan B-Virus (Sumiyoshi et al, 1992) geeignete Vektorsysteme gefunden werden konnte, um komplette cDNA-Templates herzustellen, konnte dieses Problem für Gelbfieber-Virus nur durch Klonierung von drei einzelnen DNA-Fragmenten überwunden werden (Rice et al, 1989). Im übrigen konnten für Dengue-2 und andere Flaviviren bis heute keine geeigneten Vektorsysteme gefunden werden. Die genauen Ursachen dieser Stabilitätsprobleme sind unbekannt. Aus diesem Grunde lassen sich die Ergebnisse, die bei der Herstellung eines infektiösen Klons eines bestimmten Flavivirus erhalten werden, nicht auf andere Flaviviren übertragen.

Insbesondere war es für das TBE-Virus bisher nicht bekannt, in welchem Vektorsystem, in welcher Orientierung und unter Verwendung welcher DNA-Sequenzen und Wirtszellen ein infektiöser TBEV-Klon generiert werden kann.

Aufgabe der vorliegenden Erfindung ist die Bereitstellung eines infektiösen Klons als Ausgangsmaterial zur Virus- bzw. Impfstoffproduktion.

'Infektiöser Klon' bedeutet hier, dass das Genom des Virus in Form von cDNA mit Hilfe gentechnologischer Methoden in ein Vektorsystem eingebracht wird, das es erlaubt, gezielte Veränderungen an diesem Genom vorzunehmen und durch entsprechende Methoden wieder Viren mit gegebenenfalls veränderten Eigenschaften herzustellen. Es ist bekannt, dass bei komplexen Genomen und insbesondere bei RNA-Genomen, hierbei eine Vielzahl von Problemen zu überwinden sind.

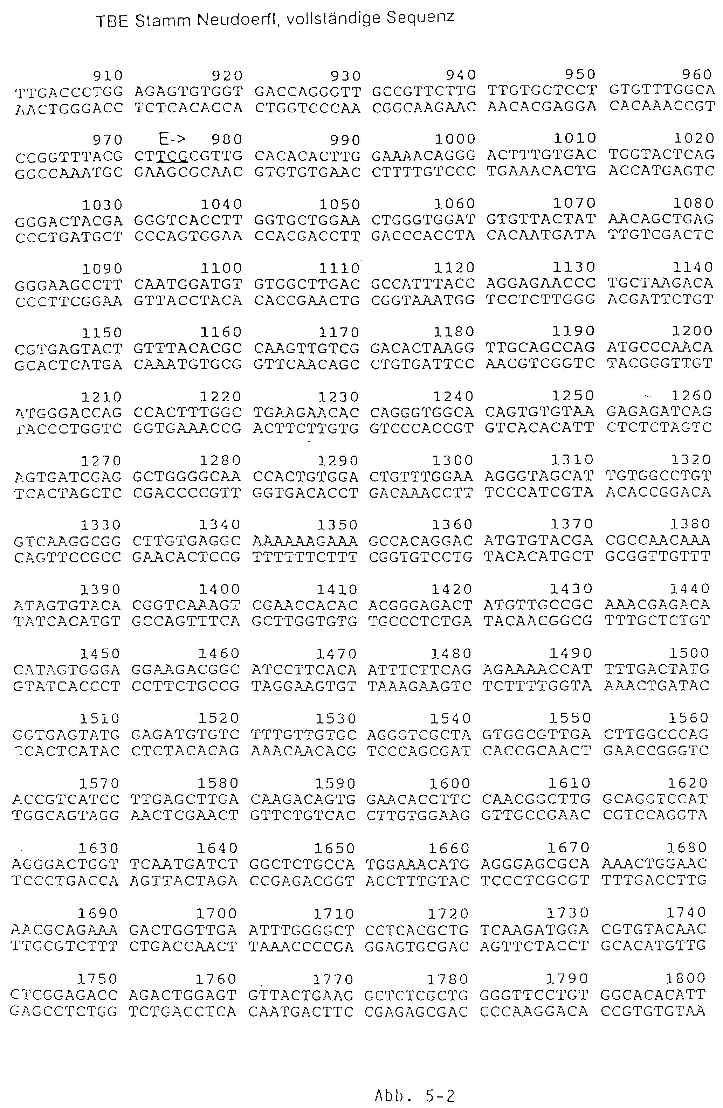

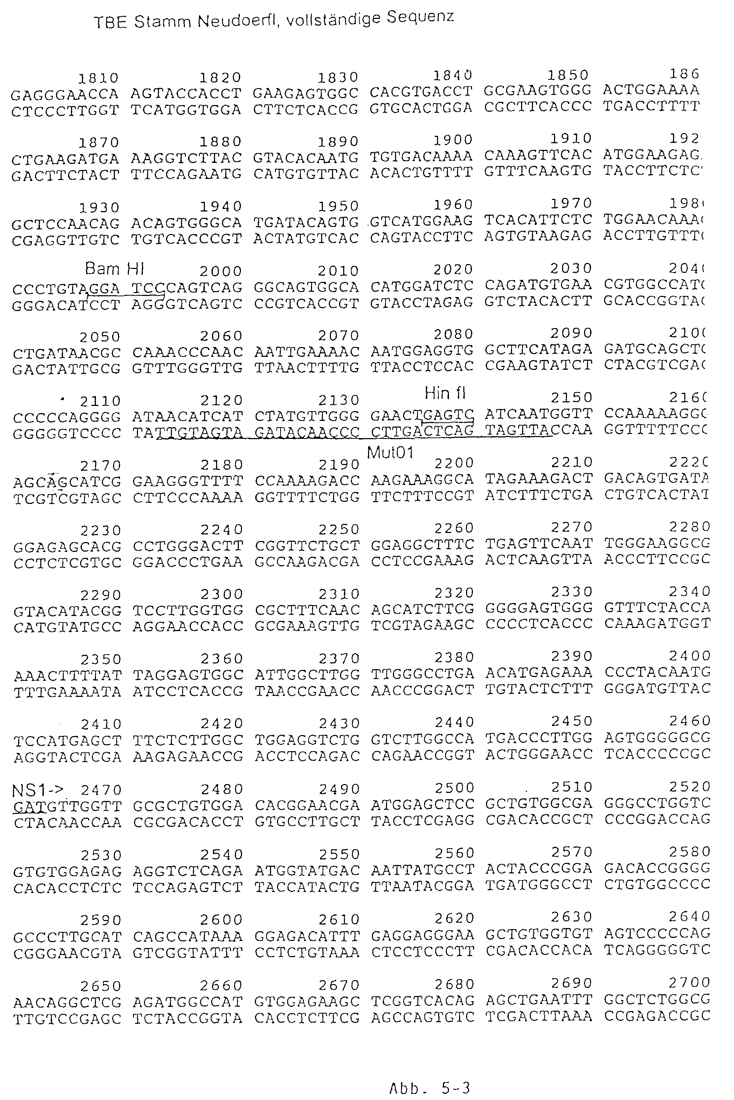

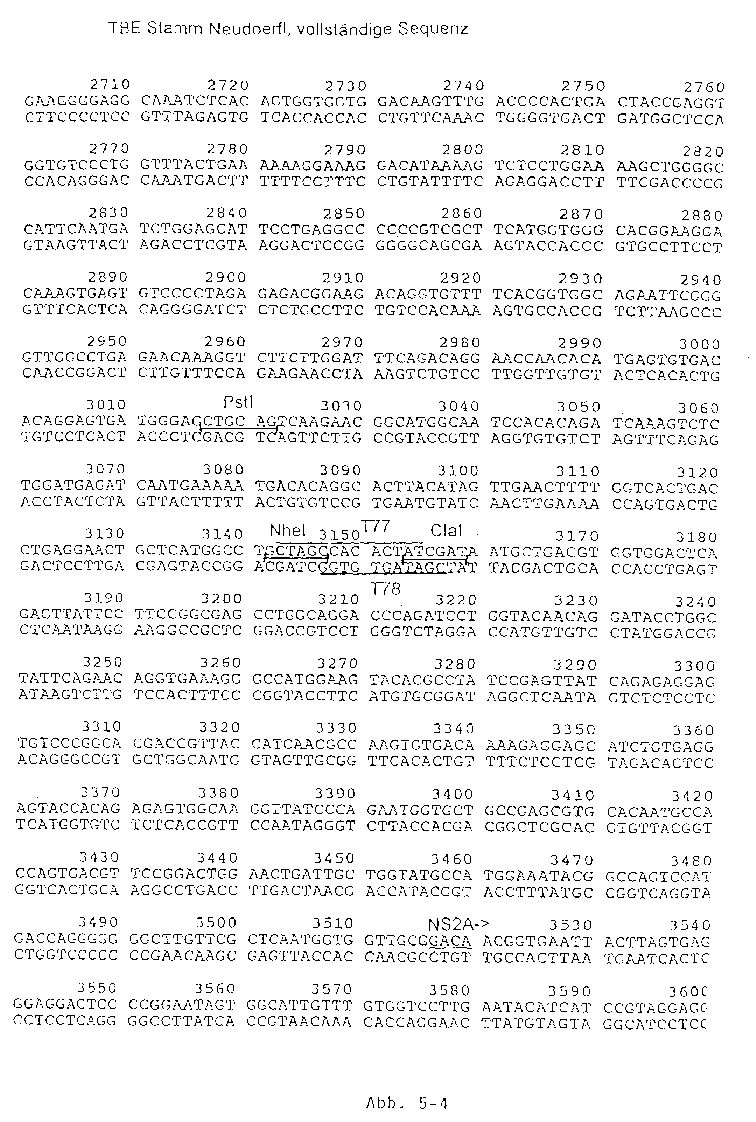

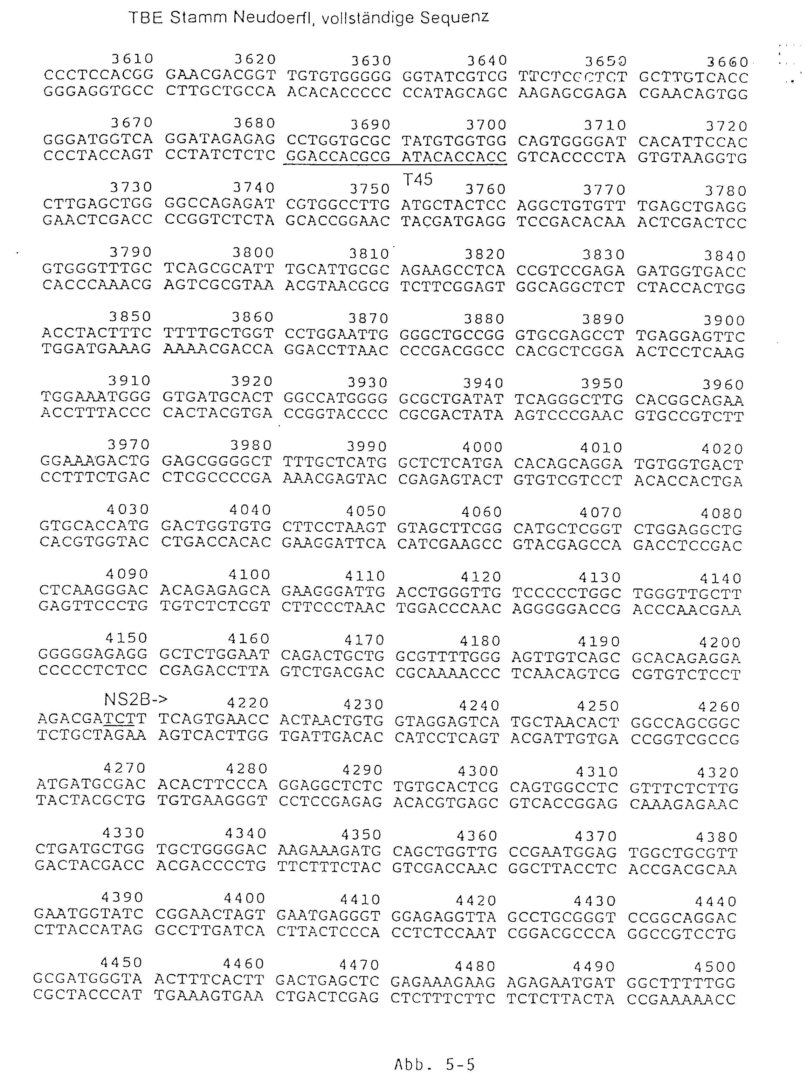

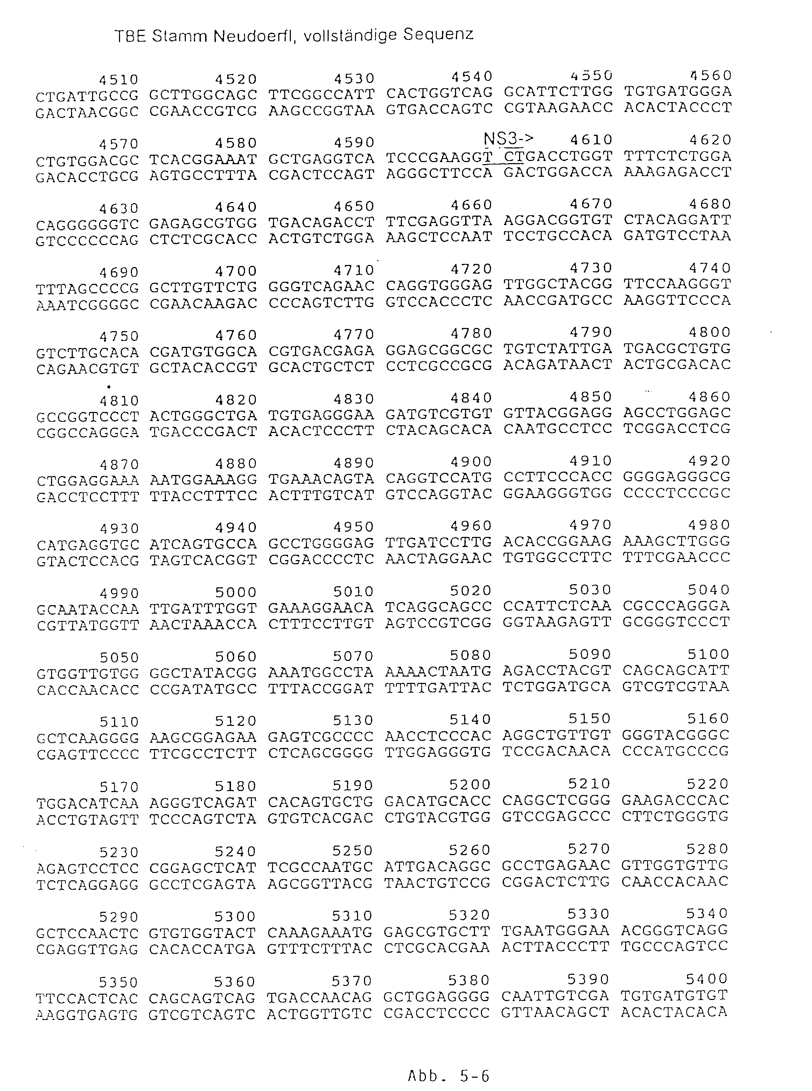

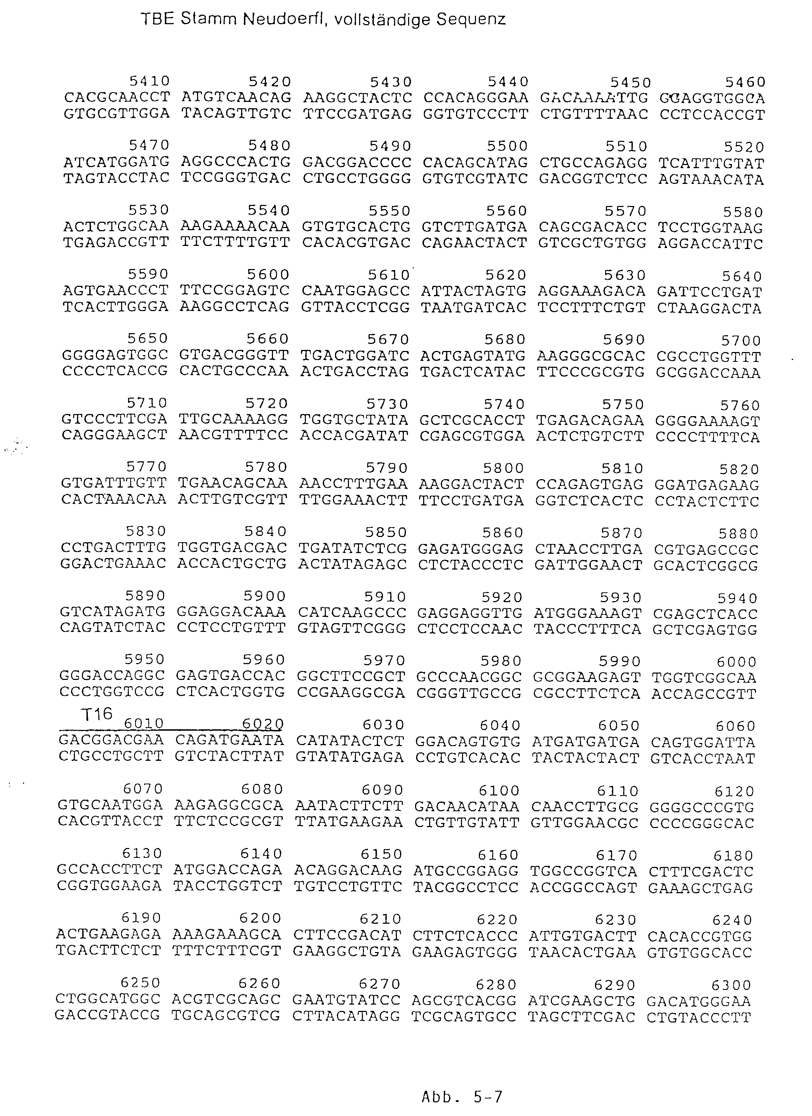

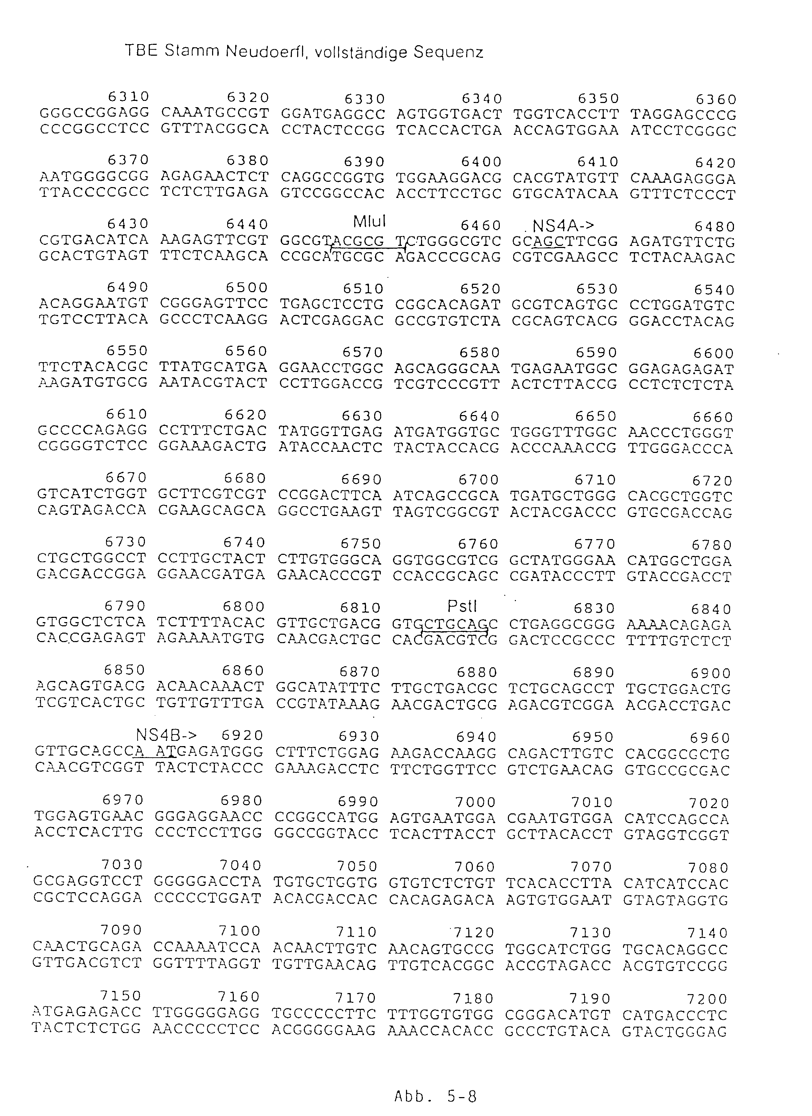

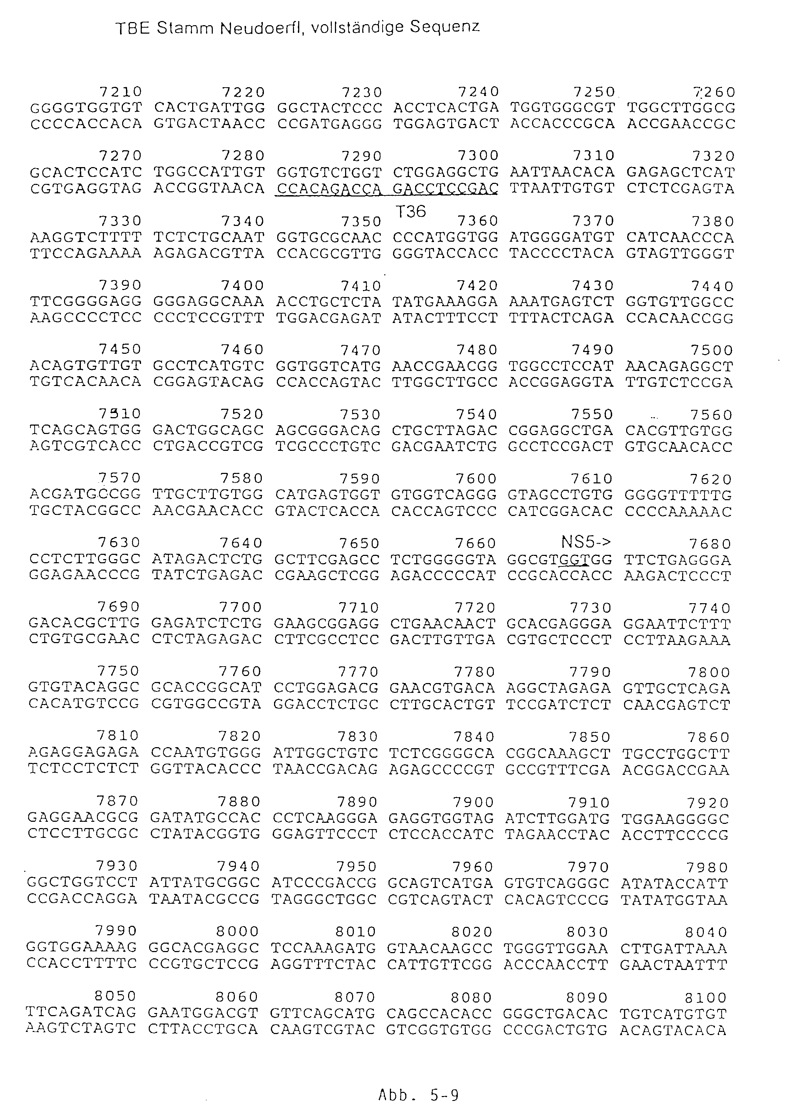

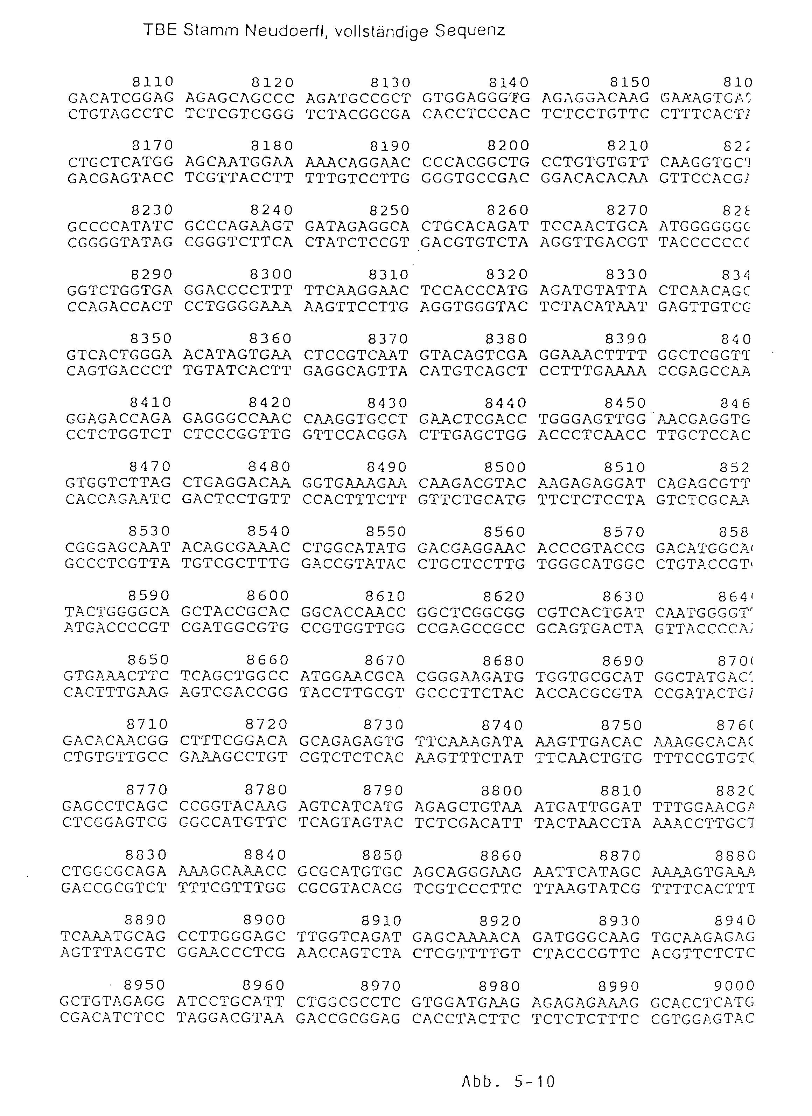

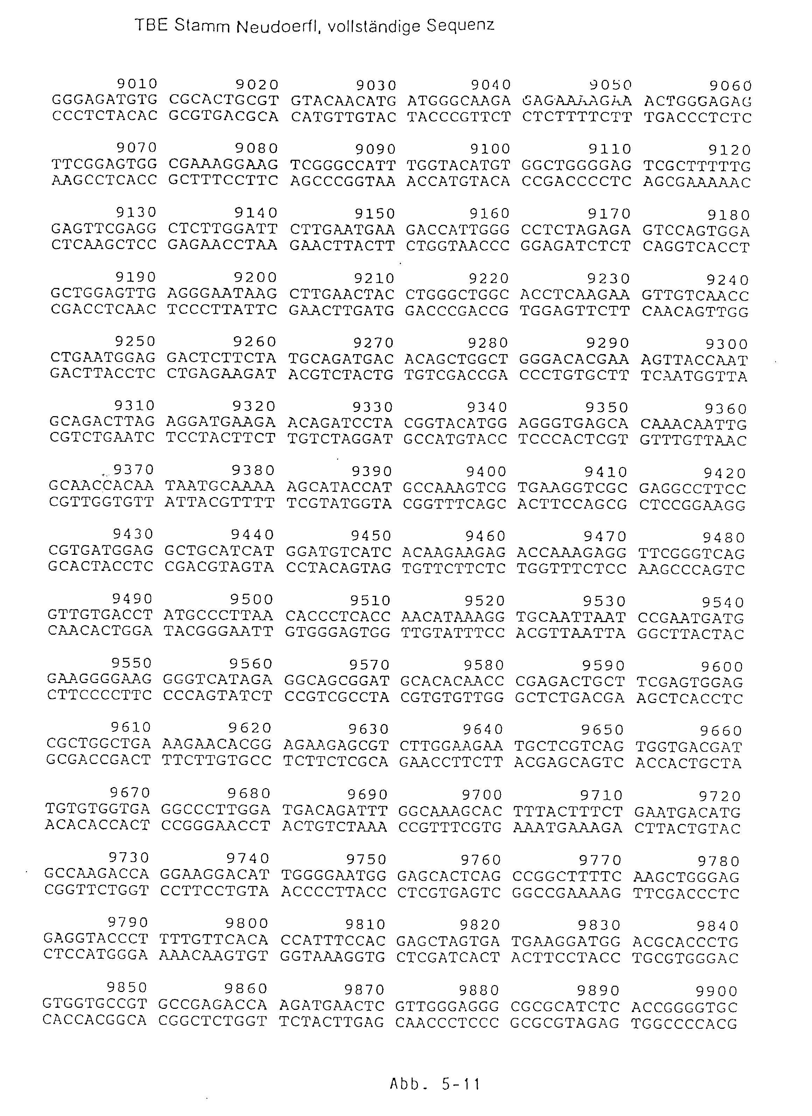

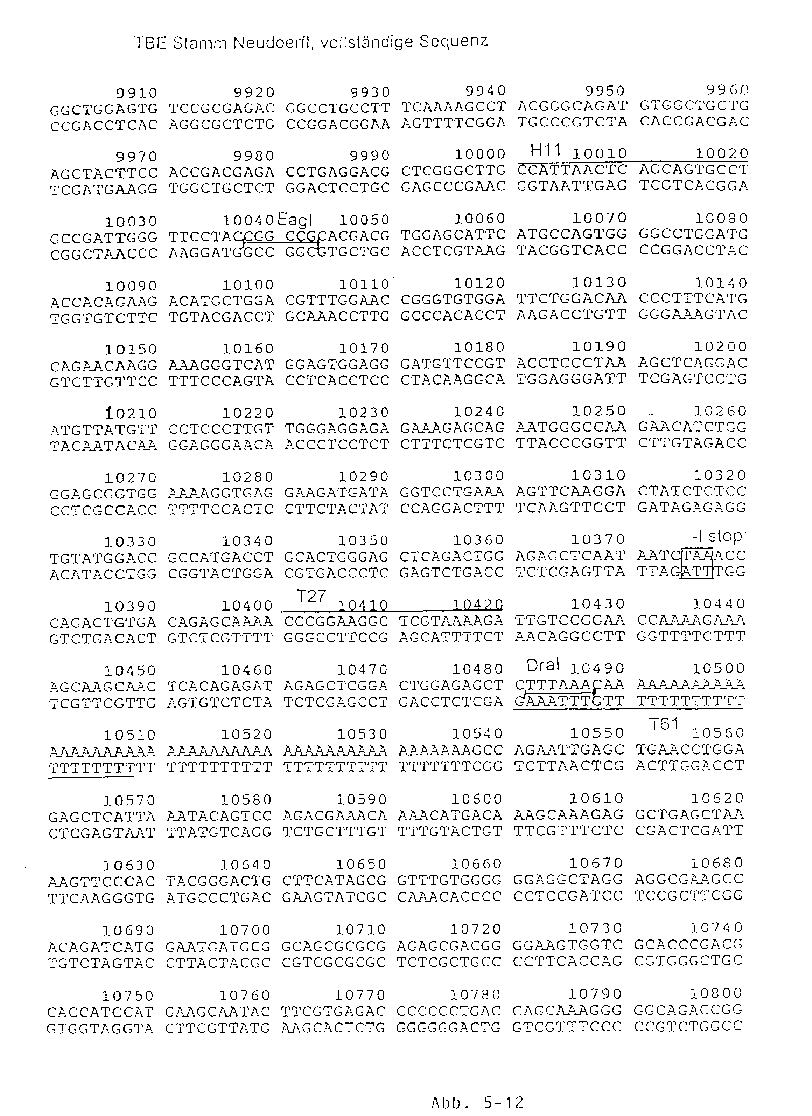

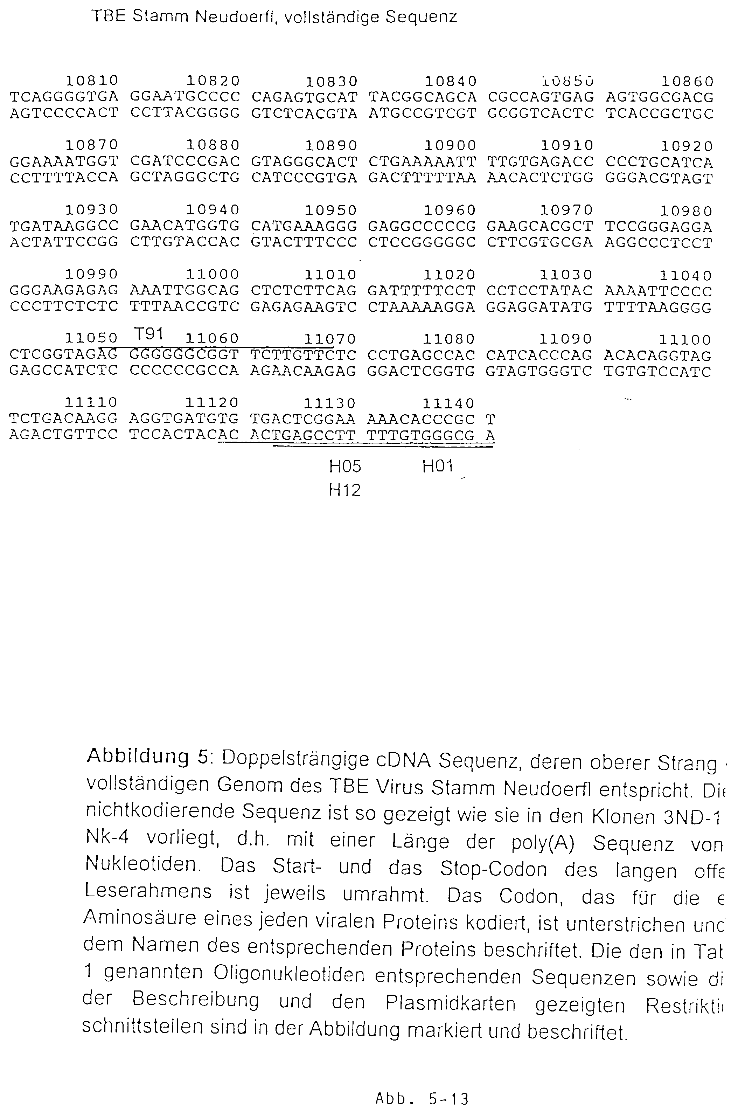

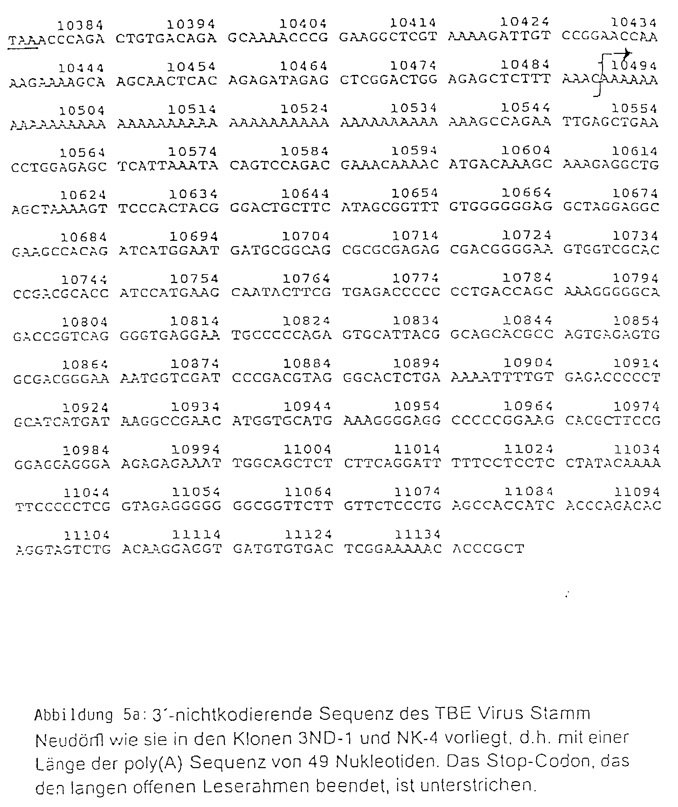

Die vorliegende Erfindung stellt erstmals eine komplette Nukleinsäure zur Verfügung die für ein infektiöses TBEV-RNA-Transkript kodiert und gekennzeichnet ist durch
a) die Sequenz gemäss Fig. 5, sowie
b) Sequenzen, die mit der Gesamtsequenz von Fig. 5 zu 70% homolog sind. Nach einer besonderen Ausführungsform beträgt diese Homologie 80 und bevorzugt 95%.

Die Nukleinsäuresequenz gemäss Fig. 5 stellt die komplette cDNA des TBE-Virus vom Stamm Neudörfl dar. Diese Gesamtsequenz einschliesslich der 5'- und 3'-nicht-kodierenden Nukleotidsequenz dieses Stammes bildet eine wesentliche Voraussetzung für das erfindungsgemässe Verfahren zur Gewinnung eines infektiösen TBEV-Klons. Die im Stand der Technik bisher veröffentlichte Sequenz umfasste lediglich eine Teilsequenz, welche bis zum Nukleotid 10488 reichte. Die Sequenz, beginnend mit Poly(A) an Position 10489 ist neu und komplettiert erstmals die Nukleinsäuresequenz, welche für ein infektiöses TBEV kodiert.

Die schnelle Entwicklung gentechnologischer Methoden der letzten Jahre hat es in zunehmendem Masse möglich gemacht, Nukleinsäuren gezielt zu verändern. Die bekannten Methoden der gezielten genetischen Veränderung beschränken sich aber auf doppelsträngige DNA-Moleküle. Viren mit relativ kleinen Genomen aus doppelsträngiger DNA, wie beispielsweise die Polyomaviren, konnten deshalb schon sehr bald gezielt genetisch verändert werden: Das Genom wurde direkt in ein geeignetes bakterielles Vektorsystem kloniert, verändert und in seiner veränderten Form in Wirtszellen gebracht, wo es zur Bildung der gewünschten Virusmutante führte (Shah, 1990).

Im Gegensatz zu DNA-Viren müssen bei RNA-Viren wesentlich aufwendigere Wege beschritten werden. Zunächst muss eine doppelsträngige Kopie (cDNA) des RNA-Genoms hergestellt werden. Diese cDNA wird dann als Ganzes oder in Fragmenten in geeigneten bakteriellen Systemen kloniert, analysiert und gezielt verändert. Von dieser DNA wird dann in vitro oder in einer Zelle eine positiv-strängige RNA-Kopie generiert, die zur Bildung des gewünschten, gezielt abgewandelten Virus führt. Dabei ist Voraussetzung, dass ein solcher infektiöser Klon alle genetischen Elemente, die für die Replikation des Virus notwendig sind, umfasst. Das Fehlen eines einzigen Nukleotids oder der Einbau eines einzigen falschen Nukleotids kann dazu führen, dass kein infektiöses Virus generiert wird. Erstmals ist es auf der Basis der in Fig. 5 zur Verfügung gestellten DNA-Sequenz des Stammes Neudörfl möglich, zu einem infektiösen Klon zu gelangen, der es ermöglicht, das Genom eines TBE-Virus gezielt zu verändern. Dies erfolgt, indem die klonierte cDNA in infektiöse RNA überführt wird. Dieser Vorgang kann entweder in der Zelle oder in vitro stattfinden. Es ist dabei erforderlich, dass dieser Transkriptionsschritt unter der Kontrolle eines geeigneten Promotor/Enzym-Systems steht, welches erlaubt, den gesamten cDNA-Klon in RNA zu übersetzen. Die häufig verwendeten RNA-Polymerasen, wie z.B. SP6, T3, T7, unterscheiden sich hinsichtlich ihrer Fähigkeit, lange Transkripte herzustellen; sie besitzen die Eigenschaft, die Tanskription bevorzugt an für jedes Enzym verschiedenen Stellen abzubrechen. Auch diesbezüglich war bisher nicht bekannt, welches Enzym in der Lage ist, einen cDNA-Klon des TBE-Genoms in eine vollständige RNA zu transkribieren.

Wie bereits vorstehend ausgeführt, ist es möglich, die cDNA gezielt durch Mutationen in ihrem kodierenden oder nichtkodierenden Bereich zu verändern. Auf diese Weise können die Virulenz oder die Ausbeute des Virus beeinflusst werden. Die Erfindung stellt somit für TBEV kodierende cDNA-Sequenzen zur Verfügung, die durch Substitution, Insertion oder Deletion so verändert sind, dass ein in seiner Virulenz vermindertes TBEV produziert wird, oder dass dadurch die Replikationsrate von TBEV beeinflusst wird.

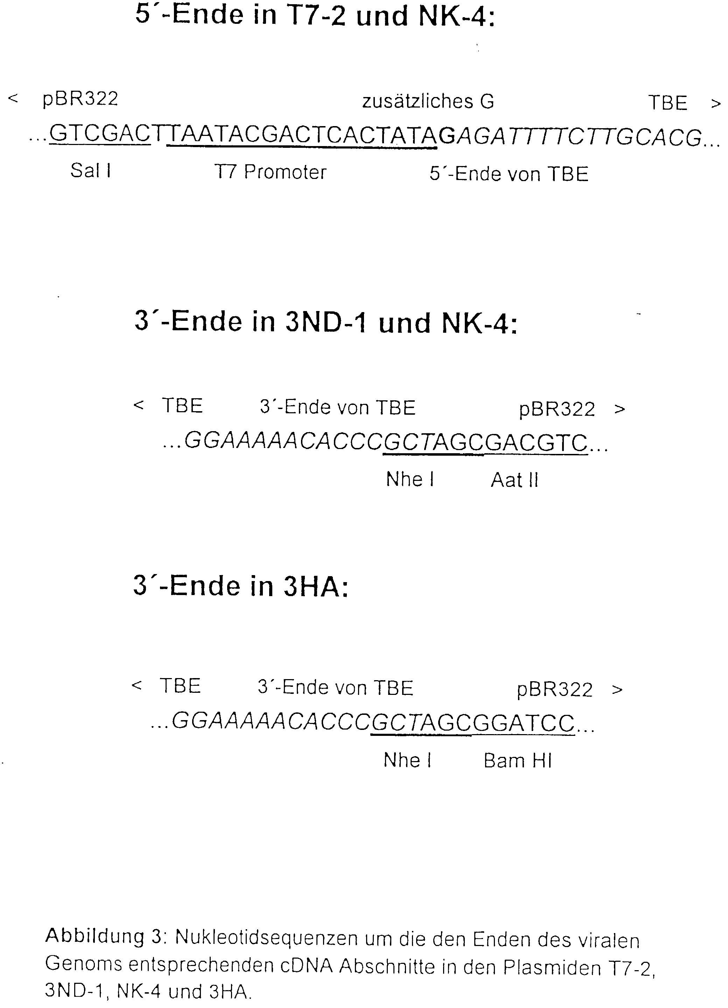

Nach einer besonderen Ausführungsform wird eine cDNA zur Verfügung gestellt, die sich durch eine Mutation am 5'-Ende auszeichnet. Das 5'-Ende der Flaviviren trägt eine CAP-Struktur am ersten Nukleotid, einem A (Mandl et al, 1989). Eine solche Struktur wird mit den gängigen RNA-Polymerasen nur mit schlechter Effizienz gebildet. Erfindungsgemäss wird die cDNA gemäss Fig. 5 so verändert (Fig. 3), dass das CAP an ein 5'-terminales G angehängt wird. Auf diese Weise entsteht eine RNA, die gegenüber der nativen viralen RNA um ein Nukleotid verlängert ist. Diese gezielte Veränderung der cDNA führt dazu, dass infektiöse RNA in wesentlich höheren Ausbeuten gebildet wird.

Weitere Modifizierungen der für TBEV kodierenden cDNA können an der pr(M)-Spaltstelle durchgeführt werden. Die Spaltung des pr(M)-Proteins zu dem in reifen Virionen vorhandenen M-Protein ist für die Infektiosität des TBE-Virus notwendig.

Verminderung der Effizienz dieser Spaltung führt zu einer Attenuierung des Virus.

Eine weitere Mutation, die zu einer herabgesetzten Virulenz von TBEV führt, besteht in der Entfernung einer Glykosylierungsstelle, wie z.B. durch Mutation an der entsprechenden N-Glykosylierungsstelle des E-Proteins.

Eine weitere Mutation kann durch Veränderung der Aminosäure 384 des E-Proteins erfolgen, wie dies z.B. in der Mutante B4 (Holzmann et al, 1990) vorliegt.

Ein weiteres attenuierendes Prinzip für TBE-Virus besteht im Einbau von einer oder mehreren Mutationen, entsprechend der Sequenz des TBE-Virusisolats 263. Bei diesem Stamm handelt es sich um ein natürlich vorkommendes TBE-Virus mit attenuierten Eigenschaften, dessen Genomsequenz sich an den in Tabelle 3 gezeigten Stellen von der des TBE-Virus Stamm Neudörfl unterscheidet.

Durch Mutationen an der für TBEV kodierenden cDNA, wie sie beispielhat oben angeführt werden, ist es möglich, ein attenuiertes Virus zu gewinnen, welches sich als Lebendimpfstoff eignet. Durch die Kombination von mindestens zwei attenuierenden Prinzipien kann der Grad der Attenuierung auf das gewünschte Mass erhöht und die Sicherheit multiplikativ erhöht werden (die Wahrscheinlichkeit der Reversion zum virulenten Wildtyp bei zwei unabhängigen attenuierten Mutationen entspricht dem Produkt der Einzelwahrscheinlichkeiten). Die Reversionshäufigkeit von einzelnen Aminosäuremutationen kann durch Ausnutzung der degenerierten Natur des genetischen Codes durch Veränderung von zwei Basen der entsprechenden Codons stark eingeschränkt werden.

Die Erfindung stellt somit die Möglichkeit zur Verfügung, anstelle von zufällig aus der Natur isolierten attenuierten Virusstämmen, das molekulare Prinzip der Attenuierung, ihr Ausmass und die Wahrscheinlichkeit der Reversion zum virulenten Wildtyp gezielt zu beeinflussen. Sie stellt damit erstmals TBEV-Mutanten zur Verfügung, die als Lebendimpfstoff geeignet sind.

Darüber hinaus ist es aber gemäss der Erfindung auch möglich, die terminalen nicht-kodierenden (NC) Regionen des TBE-Virus, gezielt zu beeinflussen. Diese nicht-kodierenden Genomabschnitte viraler RNA enthalten meist besonders wichtige regulatorische Elemente. Diese Elemente bestimmen die Replikation des viralen Genoms, beeinflussen die Bildung viraler Proteine und sind wahrscheinlich erforderlich, um die Verpackung viraler Genome in Virionen zu gewährleisten. Die biologischen Eigenschaften eines Virus, einschliesslich seiner Virulenz und Pathogenität werden somit ganz entscheidend von den NC-Genomabschnitten beeinflusst. Es ist deshalb für das Funktionieren eines infektiösen TBEV von besondere Bedeutung, dass diese NC-Genomabschnitte korrekt und genetisch stabil in die für TBEV kodierende cDNA eingebaut werden.

Der Vergleich etlicher Stechmücken-übertragener Flaviviren ergab, dass sowohl die 5'- als auch die 3'-terminalen NC-Genomabschnitte bestimmte zwischen diesen Viren konservierte Sequenzelemente enthalten. Die Analyse der Genomsequenzen von TBE-Viren zeigt in diesen Bereichen aber grosse Abweichungen von den Verhältnissen, wie sie für Stechmücken-übertragene Viren gefunden wurden. Diese Abweichungen können wie folgt zusammengefasst werden:
i) 5'-terminale Sekundärstrukturen sind innerhalb der TBE-Viren nicht konserviert und unterscheiden sich von den konservierten Sekundärstrukturen von Steckmückenübertragenen Viren;
ii) während Stechmücken-übertragene Flaviviren ein zweites Startcodon aufweisen, kommt ein solches bei TBE-Viren nicht vor;
iii) während Stechmücken-übertragene Flaviviren in der 3'-NC-Region konservierte Sequenzmotive aufweisen, fehlen diese bei TBE-Viren völlig;
iv) eine potentielle Zirkularisierungssequenz der Stechmücken-übertragenen Flaviviren hat kein Homolog bei TBE-Viren.
v) Die 3'-NC-Region des TBE-Virus zeigt selbst bei nahe verwandten Stämmen eine ausserordentliche Variabilität, wie sie sonst bei keinem anderen Flavivirus gefunden wurde. So besitzt z.B. der Stamm Neudörfl eine fast doppelt so lange 3'-NC-Region (siehe Fig. 5a) wie der Stamm Hypr. Ersterer weist Sequenzelemente auf, die bei anderen Stämmen und anderen Flaviviren nicht vorkommen. Eines dieser Elemente ist ein poly(A)-Motiv, dessen Länge zwischen 6 As und mehreren hundert As variieren kann. Eine derartige Struktur wurde bei Steckmückenübertragenen Flaviviren nie aufgefunden.

Die Erfindung umfasst die erstmals zur Verfügung gestellte 3'-NC-Region des Stammes Neudörfl, wie sie in Fig. 5a wiedergegeben ist, sowie solche 3'-NC-Sequenzen, die der von Fig. 5a zu 65%, vorzugsweise zu 80% homolog sind. Der Verwendung dieser 3'-NC-Sequenzen kommt besondere Bedeutung zu für die Herstellung von Impfstoffen gegen Varianten und Subtypen von TBEV.

Das TBEV kommt in der Natur bekanntlich in verschiedenen Varianten vor. Insbesondere findet man im Osten Russlands und in China ein als fernöstlicher (FE) Subtyp bezeichnetes TBE-Virus, das mit dem Auftreten eines wesentlich stärkeren Krankheitsbildes mit höherer Morbidität und Letalität verbunden ist. Andererseits weisen diese Subtypen, wie auch viele andere Stämme des TBE-Virus sowie nahe verwandete andere TBE-Viren (z.B. Looping III, Turkish Encephalitis Virus, Omsk Hemorrhagic Fever Virus) mehr oder weniger starke Abweichungen ihrer Antigenstrukturen von jenen des Stammes Neudörfl auf. Für eine optimale protektive Wirkung sollten aber die antigenen Eigenschaften eines Impfstoffes mit jenen der in der entsprechenden Region zirkulierenden Viren möglichst übereinstimmen. Da diese Stämme aber teilweise unterschiedliche Virulenzeigenschaften aufweisen und sich im Aufbau ihrer 3'-NC-Region signifikant voneinander unterscheiden können, ist es problematisch, für jeden dieser Stämme attenuierte Impfstoffe auf der Basis spezifischer eigener infektiöser Klone herzustellen.

Durch die vorliegende Erfindung ist es möglich, Sequenzen, die für Proteine von verschiedenen Stämmen, Subtypen oder nahen verwandten TBE-Viren kodieren, mit der 3'-NC-Region des Stammes Neudörfl gemäss Fig. 5a oder einer leicht davon abgewandelten Sequenz, zu kombinieren. Auf diese Weise gelingt es, attenuierte Impfstoffe mit den gewünschten antigenen Eigenschaften herzustellen. Die Erfindung umfasst somit für ein infektiöses TBEV kodierende Nukleinsäuren, welche neben der kodierenden Sequenz eines beliebigen TBEV-Stammes oder Subtyps die 3'-nicht-kodierende Sequenz gemäss Fig. 5a umfassen.

Ausserdem sollen gemäss der Erfindung auch für infektiöses TBEV kodierende Nukleinsäuren umfasst sein, die sich aus der kodierenden Sequenz nach Fig. 5 und einer 3'-nicht-kodierenden Sequenz eines verwandten oder eines anderen TBEV oder Flavivirus zusammensetzen. Besonders bevorzugt sind solche Konstrukte, die die TBEV-kodierende Sequenz nach Fig. 5 und die 3'-nicht-kodierende Sequenz des TBEV-Stammes Hypr umfassen.

Die Erfindung schliesst sämtliche infektiösen TBEV-Klone mit ein, die sich als RNA-Transkript aus den vorstehend genannten Sequenzen ableiten.

Im weiteren umfasst die Erfindung solche cDNA-Konstrukte, die durch Einbau einer für einen Immunmodulator kodierenden Nukleotidsequenz zu einem infektiösen Klon mit einer effektiveren Immunantwort führen. Aus gesundheitspolitischen, organisatorischen und kostentechnischen Gründen ist es wünschenswert, dass ein Impfstoff eine lang anhaltende und breite Immunantwort induziert. Bei vielen derzeit in Verwendung stehenden Vakzinen sind häufige Auffrisch-Impfungen nötig, und die Immunisierung gegen verschiedene Viren kann nicht durch einen einzigen Impfstoff erfolgen. In die 3'-NC-Region können zusätzliche genetische Informationen eingebracht werden, wodurch die Immunantwort moduliert oder stimuliert werden kann. Dies ist auch möglich, wenn man Teile der 3'-NC-Region durch entsprechende, für einen Immunmodulator kodierende Sequenzen ersetzt. Derartige, für einen Immunmodulator kodierende Sequenzen können ausgewählt werden aus DNA-Sequenzen, die für Interleukine, Diphtherietoxin, Choleratoxin, E. coli-hitzelabilem Toxin und Pertussistoxin kodieren. Auf diese Weise kann die Effizienz einer Vakzine erhöht und/oder eine länger wirkende Immunantwort erzielt bzw. die Breite der Immunantwort (z.B. gegen mehrere Varianten des TBE-Virus) erweitert werden.

Die Herstellung des erfindungsgemäss geschützten infektiösen TBEV-Klons wird durch die nachfolgende Beschreibung sowie die Ausführungsbeispiele und die sich anschliessenden Abbildungen und Tabellen näher erläutert:
- Abb. 1: zeigt die Plasmidkarte des Vektorplasmids pBR322 mit für die Konstruktion der beschriebenen Klone relevanten Restriktionsschnittstellen.
- Abb. 2: zeigt die Plasmidkarte des Klons T7-2 mit den relevanten Restriktionsschnittstellen.
- Abb. 3: zeigt die Nukleotidsequenzen der 5'- und 3'-terminalen Enden der Klone T7-2, NK-4, 3ND-1 und 3HA.

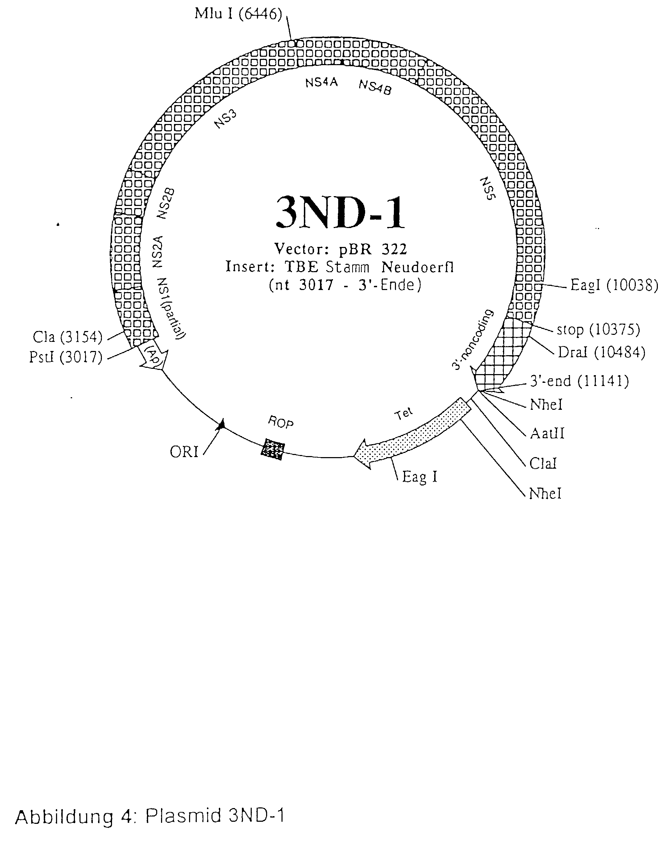

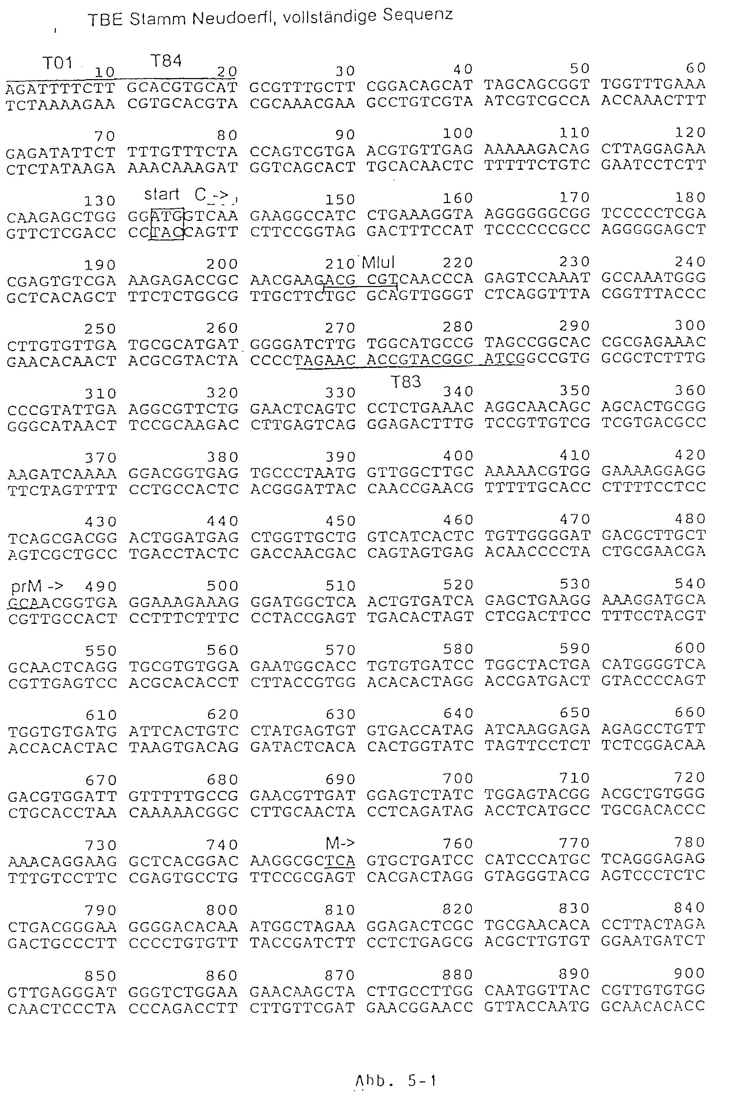

- Abb. 4: zeigt die Plasmidkarte des Klons 3ND-1, mit den relevanten Restriktionsschnittstellen.
- Abb. 5: zeigt die doppelsträngige komplette cDNA-Sequenz des TBEV Stamm Neudörfl Genoms einschliesslich der 3'-nicht-kodierenden Sequenz, wie sie in den Klonen 3ND-1 und NK-4 mit einer poly(A)-Länge von 49 Nukleotiden vorliegt. Das Stop-Codon, das den offenen Leserahmen terminiert, ist unterstrichen.
- Abb. 5a: gibt die 3'-nicht-kodierende Sequenz von TBEV Stamm Neudörfl wieder.

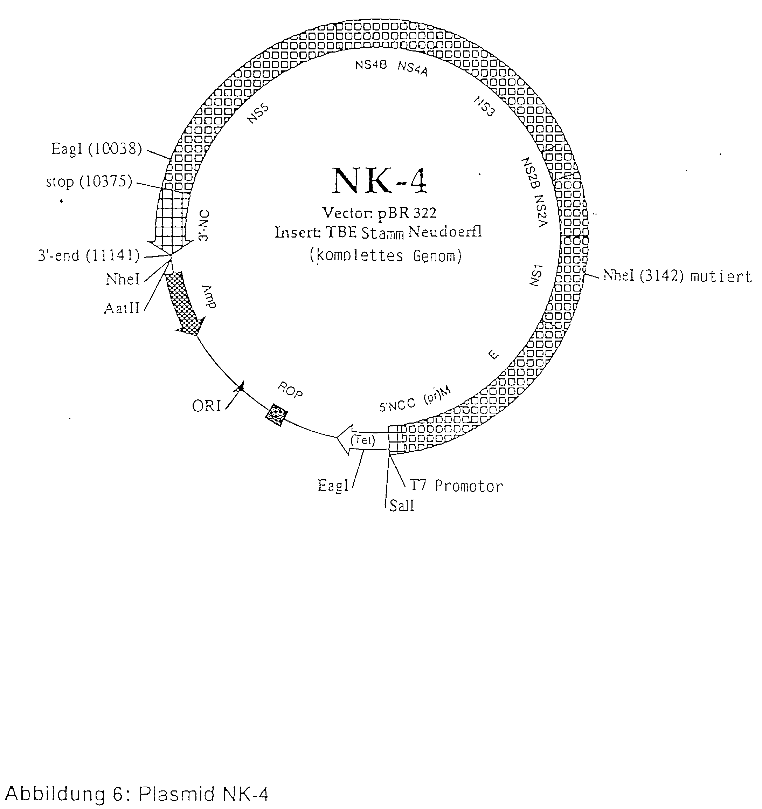

- Abb. 6: zeigt die Plasmidkarte des Klons NK-4 mit den relevanten Restriktionsschnittstellen.
- Abb. 7: zeigt die Plasmidkarte des Klons 3HA mit den relevanten Restriktionsschnittstellen.
- Abb. 8: zeigt einen Vergleich der Neurovirulenz und Neuroinvasivität zwischen TBE-Virus Stamm Neudörfl und der Mutante RB4.
- Tabelle 1: zeigt eine Liste der für die Herstellung der cDNA aus TBE-Virus-RNA und die Inserierung von Oligonukleotiden verwendeten Primern und deren Position auf der TBEV-Nukleotidsequenz entsprechend Abb. 5.
- Tabelle 2: zeigt die Sequenzunterschiede zwischen dem hergestellten infektiösen Klon von TBEV und der wt-Sequenz von TBE-Virus Stamm Neudörfl. Durch Sequenzanalyse der cDNA des infektiösen Klons von TBEV und der wt-DNA-Sequenz des Stammes Neudörfl konnten einige Sequenzänderungen, die bei der Herstellung der IK auftraten, identifiziert werden.
- Tabelle 3: zeigt Sequenzunterschiede zwischen TBE-Virus Stamm Neudörfl und dem attenuierten natürlichen Isolat 263.
- Tabelle 3a: gibt noch einmal in übersichtlicher Form Modifikationen und deren Position an, die erfindungsgemäss angaewandt werden können, um zu einem atenuierten Stamm zu gelangen.

Die erfindungsgemässe Herstellung von TBE-Virus mit Hilfe des infektiösen TBEV-Klons kann mit Hilfe folgender Verfahrensschritte erfolgen:
a) In vitro Herstellung eines RNA-Transkripts aus einem rekombinanten DNA-Konstrukt, welches die DNA gemäss Fig. 5 umfasst,
b) Transfektion von Wirtszellen mit dem unter a) erhaltenen RNA-Transkript, und
c) Gewinnung infektiöser Viruspartikel.

Alternativ kann die Transkription der RNA auch in vivo (also in Wirtszellen) durchgeführt werden:
a) Transfektion von Wirtszellen mit einem DNA-Konstrukt, welches die DNA von Fig. 5 umfasst, und
b) Gewinnung infektiöser Viruspartikel.

In den vorstehend genannten Verfahren bedient man sich zur Transformation bzw. Transfektion der Wirtszellen eines DNA-Konstruktes, welches neben den für TBEV kodierenden DNA-Sequenzen ein Vektorsystem sowie einen Promotor umfasst. Geeignete Promotoren werden ausgewählt aus den prokaryontischen Promotoren SP6, T3 und T7 oder aus den eukaryontischen Promotoren von CMV, RSV, ss-Aktin, SV40 oder Adeno-2.

Sofern ein in seiner Virulenz veränderter TBEV-Klon hergestellt oder die Ausbeute an infektiösem Virus durch Veränderung des TBEV-Genoms verbessert werden soll, ist es erforderlich, von einer, wie vorstehend ausgeführt, modifizierten DNA auszugehen. Die entsprechenden Herstellungsschritte für die Gewinnung von TBE-Virus mit Hilfe eines infektiösen Klons umfassen dann entweder
a) die in vitro Herstellung eines RNA-Transkripts von einem rekombinanten modifizierten DNA-Konstrukt,
b) die Transfektion von Wirtszellen mit dem viralen RNA-Transkript, und
c) die Gewinnung infektiöser modifizierter Viruspartikel, oder
a1) die Transfektion einer Wirtszelle mit einem rekombinanten modifizierter DNA-Konstrukt, und
b1) die Gewinnung infektiöser modifizierter Viruspartikel.

Die vorstehend aufgeführten alternativen Verfahren zur Herstellung von TBE-Virus mit Hilfe eines infektiösen TBEV-Klons erfolgen über transformierte oder transfizierte prokaryontische oder eukaryontische Wirtszellen, die ebenfalls von der Erfindung umfasst sein sollen. Als besonders bevorzugte Wirtszellen eignen sich E. coli, Vero-, CHO- oder BHK-Zellen.

Die vorliegende Erfindung umfasst ausserdem die Bereitstellung eines Impfstoffes gegen Zecken-übertragene Enzephalitis, welcher ein rekombinant hergestelltes TBE-Virus zusammen mit einem pharmazeutisch akzeptablen Träger und gegebenenfalls einen Immunmodulator enthält.

Sofern ein Totimpfstoff hergestellt werden soll, geht man von einem infektiösen TBEV aus, das, wie oben beschrieben, inaktiviert wird. Bevorzugt geht man von einem attenuierten TBE-Virus aus. So kann z.B. mit Hilfe des erfindungsgemässen infektiösen Klons ein attenuierter Stamm hergestellt werden, der als Saatvirus für die Produktion von Totimpfstoff eingesetzt werden kann. Ein solches Saatvirus mit abgeschwächter Pathogenität reduziert das Sicherheitsrisiko, das mit der Produktion von Totimpfstoffen verbunden ist. Wie vorstehend ausgeführt, ist es dabei wünschenswert, dass der verwendete Virusstamm in entsprechenden Zellkulturen möglichst effizient vermehrt wird, um die Ausbeute an Virus zu maximieren. Eine erhöhte Ausbeute wird erfindungsgemäss gegebenenfalls durch Einbringung von Mutationen in der 3'-nicht-kodierenden Region gemäss Fig.5a erhalten. Durch gleichzeitige Mutation an anderen Stellen des Genoms ist es möglich, Stämme herzustellen, die bei gleichen Produktionskosten zu höheren Ausbeuten führen, aber in ihrer Virulenz gegenüber dem Stamm Neudörfl vermindert sind.

Die Herstellung des Impfstoffes erfolgt nach dem Fachmann bekannten Verfahren. Der Impfstoff enthält pro Einheitsdosierung eine sichere und immunologisch effektive Menge an rekombinantem Virus und einen pharmazeutisch akzeptablen Träger sowie gegebenenfalls einen Immunmodulator.

Für die Herstellung von Lebendimpfstoff ist es besonders bevorzugt, von einem attenuierten TBE-Virus auszugehen. Die Dosis liegt dabei bevorzugt im Bereich von 1 - 10.000 infektiösen Einheiten pro Erwachsenem, besonders bevorzugt bei 10 - 100 infektiösen Einheiten.

Diese Impfung kann nicht nur mit dem von den erfindungsgemässen Nukleinsäuren abgeleiteten Virus erfolgen, sondern auch durch direkte Verabreichung der Nukleinsäuren selbst.

Dabei produzieren die Zellen des geimpften Organismus von den verabreichten Nukleinsäuren jene attenuierten Viren, die eine Immunantwort auslösen. Die direkte Verwendung der erfindungsgemässen infektiösen Nukleinsäuren bietet somit den grossen Vorteil, dass für die Impfstoffherstellung im Gegensatz zu bisherigen Lebendimpfstoffen die aufwendige Virusvermehrung, beispielsweise in Zellkultursystemen, überflüssig ist. Darüber hinaus werden dadurch Probleme von konventionellen Lebendimpfstoffen, wie z.B. Lagerstabilität, Produktionssicherheit und Reinheit, gelöst.

Erfindungsgemäss ist vorgesehen, den Impfstoff für die parenterale Verabreichung, insbesondere die intramuskuläre Verabreichung zu formulieren. Daneben ist es aber auch möglich, den Impfstoff in oral verabreichbarer Form bereitzustellen.

Die Erfindung wird anhand der folgenden Beispiele näher erläutert.

### Beispiel 1

### Herstellung von cDNA

TBE-Virus wurde nach der schon früher beschriebenen Methode (Heinz und Kunz, 1981) in Hühnerembryofibroblasten-Kulturen gezüchtet und aus dem Überstand gereinigt. Diese Reinigung umfasst im wesentlichen eine Pelletierung des Virus durch Ultrazentrifugation und anschliessend eine zweimalige Auftrennung durch Zentrifugation über Sucrose-Dichtegradienten.

Aus gereinigten Viruspräparationen wurde dann die virale genomische RNA durch zweimalige Extraktion mit Phenol und Chloroform gewonnen und in Gegenwart von RNase-Inhibitor (human placental RNAsin; Promega) mit Ethanol gefällt. Die Menge und Integrität der so gewonnen RNA wurde durch Elektrophorese auf denaturierenden Glyoxal-Agarose-Gelen, die mit Acridin-Orange gefärbt wurden (McMaster und Carmichael, 1977), bestimmt. Ungefähr 1µg solcher RNA wurde dann für die Synthese spezifischer cDNA eingesetzt.

Doppelsträngige cDNA wurde nach der Methode von Gubler und Hoffman (1983) unter Verwendung der im "cDNA Synthesis Kit" (cat. no. 1013 882) der Firma Boehringer-Mannheim enthaltenen Reagaenzien, synthetisiert. Als Primer für die cDNA Synthese dienten entweder Zufallssequenz-Hexanukleotide oder spezifische Oligonukleotide von meist 20 Basen Länge (z.B. T45, T61, H01; siehe Tabelle 1, die entsprechend der von Mandl et al. (1988 und 1989) publizierten Sequenz des TBE-Virus Prototyp Stamm Neudörfl synthetisiert worden waren (Medprobe®, Oslo, Norwegen). Die erhaltene cDNA wurde auf 1% Agarose-Gelen überprüft und entweder direkt in ein bakterielles Plasmid kloniert (siehe unten) oder in Verdünnungen von 1:100, 1:1000 oder 1:10000 als Ausgangsmaterial für die Herstellung spezifischer Fragmente mit Hilfe der Polymerase-Kettenreaktion (PCR) eingesetzt. Für die PCR wurden die früher beschriebenen Bedingungen (Mandl et al., 1989) verwendet. Allerdings wurde für die Herstellung langer (1000 bis 4500 Basenpaare) PCR Fragmente die Inkubationszeit bei 68°C, während der die enzymatische DNA Synthese stattfindet, auf bis zu 7 min verlängert. Die für die PCR verwendeten Primer waren synthetische Oligonukleotide von meist 20 Basen Länge (Medprobe®) und wurden in einer Konzentration von 100 ng/ml eingesetzt. Zur spezifischen Konstruktion der den Enden des viralen Genoms entsprechenden cDNA Moleküle und zur spezifischen Mutagenese (siehe unten) wurden auch längere Oligonukleotide (bis zu 51 Basen Länge; siehe Tabelle 1), die ausser der dem viralen Genom entsprechenden Sequenz auch andere Sequenzelemente (Restriktionsschnittstellen, Promoter, Mismatches zur Mutagenese) enthielten, verwendet. Diese Primer wurden in Konzentrationen von bis zu 1 µg/ml in der PCR eingesetzt.

### Beispiel 2:

### Klonierung von cDNA

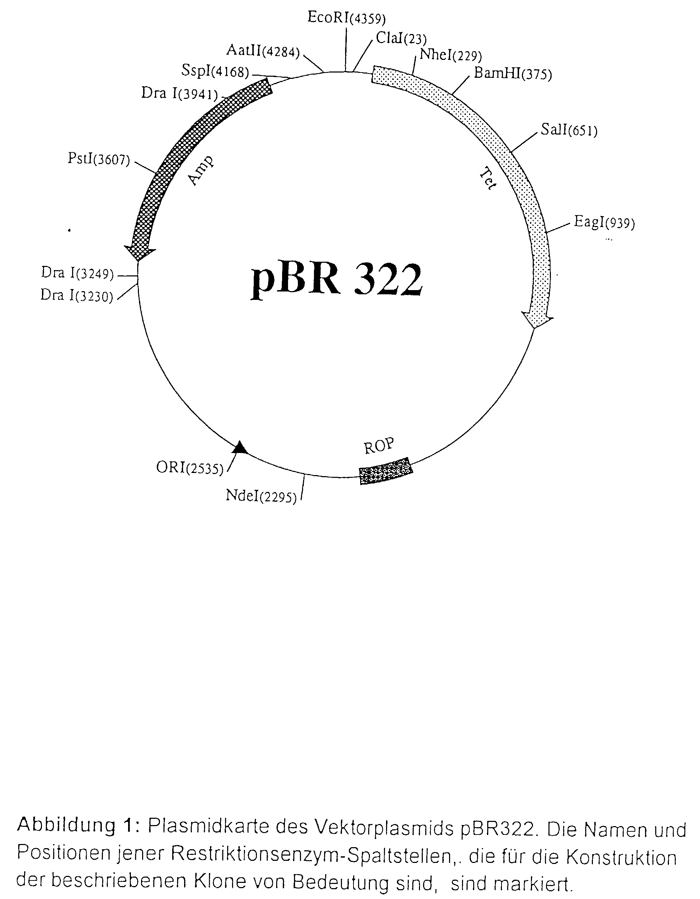

Doppelsträngige cDNA wurde mit Restriktionsenzymen, deren Schnittstellen in der Sequenz des TBE-Virus auf Grund der publizierten Genomsequenzen (Mandl et al., 1988; 1989) bekannt waren, geschnitten und in das Plasmid pBR322 (Abb. 1) kloniert. Dazu wurden 100ng pBR322 (New England Biolabs) mit demselben Restriktionsenzym geschnitten, falls nötig das entsprechende Fragment nach Elektrophorese auf 1% Agarose-Gelen (BioRad) durch Elektroelution (Elektroeluter von IBI) isoliert, und mit der cDNA gemischt. Das Gemisch wurde durch Phenol- und Chloroform-Extraktionen gereinigt, mit Ethanol gefällt und über Nacht in einem Volumen von 20 - 30 µl Ligationspuffer (Stratagene) bei 16°C mit 2µl T4 DNA Ligase (Stratagene) inkubiert. Dann wurde der Ligationsansatz bei 65°C für 5 min erwärmt um das Enzym zu inaktivieren, und 2µl von 5 bis 20-fachen Verdünnungen wurden zur Transformation von E.coli Bakterien (Stamm HB101) verwendet. Die Transformation erfolgte entweder nach der Hitzeschockmethode (Maniatis et al., 1982) unter Verwendung kompetenter Bakterien (Epicurian E.coli, Fa. Stratagene) oder durch Elektroporation mit dem GenePulser (Biorad). Rekombinante Bakterienkolonien wurden nach gängigen Standard-Labormethoden herangezüchtet und zur Isolierung von Plasmid DNA verwendet (Maniatis et al., 1982).

Die oben beschriebene Methode zur direkten Klonierung von cDNA wurde beispielsweise angewendet, um ein 3797 Basenpaar (bp)-langes cDNA Fragment (von PstI 3021 bis PstI 6818) insertiert in der singulären PstI Stelle von pBR322 zu erhalten (Klon PstI, siehe unten). Der Vorteil dieser Methode besteht darin, dass die so klonierten cDNA-Fragmente in der Regel keine Fehler in der Nukleotidsequenz aufweisen, wie das bei Verwendung der PCR häufig zu beobachten ist. Es konnten aber mit dieser Methode nicht allen Abschnitten des Genoms des TBE-Virus entsprechende cDNA-Fragmente kloniert werden. Insbesondere die Herstellung von cDNA Fragmenten, die den Enden des viralen Genoms entsprechen, erforderte den Einsatz der PCR. Fragmente, die mit PCR hergestellt worden waren, wurden anschliessend, nach derselben Methode wie oben beschrieben, kloniert oder es wurden bei der PCR Oligonukleotidprimer eingesetzt, die an ihren 5'-Enden Schnittstellen für Restriktionsenzyme aufwiesen, die dann bei der Klonierung verwendet werden konnten.

### Beispiel 3:

### Charakterisierung und Sequenzanalyse klonierter cDNA

Jedes in ein bakterielles Plasmid klonierte cDNA-Fragment wurde genau hinsichtlich seiner Lage und Orientierung im Plasmid, dem etwaigen Vorkommen von Deletionen oder Insertionen oder von durch Rekombination aufgetretenen Veränderungen untersucht. Dazu wurden einige µg Plasmid DNA aus 2-3 ml Kulturen der entsprechenden E.coli HB101 Kolonie gereinigt (Magic Mini Prep System®, Promega) und mit verschiedenen Restriktionsenzymen verdaut. Die dann auf 1 - 2% Agarose-Gelen aufgetrennten Fragmente ergaben spezifische Bandenmuster, durch die die cDNA Fragmente zunächst grob charakterisiert werden konnten. Von jenen Plasmiden, die auf Grund dieser Analysen ausgewählt wurden, wurden 1 - 2 mg in hochgereinigter Form hergestellt (CsCl-Gradienten-Reinigung, Maniatis et al., 1982). Ca 1µg solcher Plasmidpräparationen wurde dann für eine einzelne Sequenzanalysenreaktion eingesetzt. Die Sequenzanalyse erfolgte unter Verwendung Fluoreszenzfarbstoff-markierter Didesoxyterminatoren (ABI, Perkin Elmer) und des automatisierten Sequenzierautomaten 373A der Fa.ABI (jetzt Perkin Elmer) unter Anwendung der Reagaenzien dieser Firma und der vom Hersteller angegebenen Reaktionsbedingungen für die Sequenzierung doppelsträngiger DNA. Als Primer in den Sequenzreaktionen diente eine Kollektion von etwa Hundert 20mer Oligonukleotiden (Medprobe®, Oslo, Norwegen), die Sequenzen in Abständen von nicht mehr als 400 Nukleotiden auf dem Plus- und dem Minusstrang des TBE-Virus Stamm Neudörfl komplementär waren. Unter Verwendung dieser Primer konnte die Nukleotidsequenz jeder beliebigen Stelle des Genoms in beiden Orientierungen analysiert werden. Prinzipiell wurden nur Fragmente, deren Sequenz vollständig bestätigt worden war, für weiterführende Klonierungsarbeiten herangezogen. Alle PCR bedingten Mutationen, die zu Änderungen der Aminosäuresequenz geführt hätten, wurden durch Austausch von Restriktionsfragmenten mit fehlerlosen Fragmenten aus anderen Klonen, die entweder ebenfalls durch PCR oder durch direkte Klonierung von cDNA erhalten worden waren, nach Standardprotokollen (Maniatis et al., 1982) beseitigt. Die Sequenzen solcher korrigierter Klone wurde wiederum durch vollständige Sequenzanalyse überprüft.

Computeranalysen der Sequenzdaten wurden mit den Microgenie (Beckman) und PcGene (Intellegenetics) Software-Paketen durchgeführt.

### Beispiel 4:

### Konstruktion und Beschreibung von Plasmid T7-2

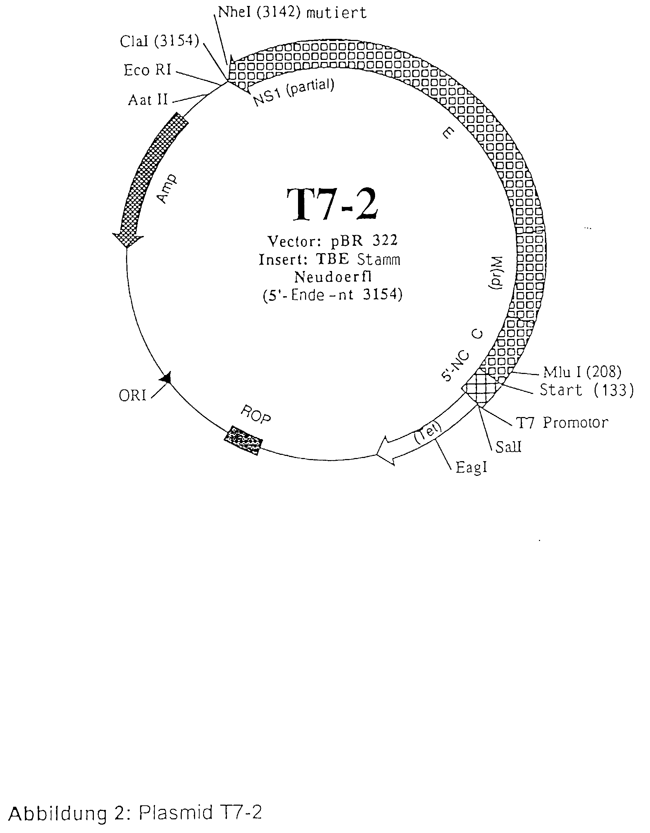

Plasmid T7-2 (Abb.2) enthält cDNA der 5'-terminalen Region des TBE-Virus Stamm Neudörfl insertiert in den Vektor pBR322 (Abb.1). In die Restriktionsschnittstellen ClaI (23) und SalI (651) von pBR322 wurde ein DNA-Fragement kodierend für TBEV-Sequenzen inseriert. Dieses Fragement umfasst das 5'-Ende (Nukleotid Position 1) bis zur in der nativen TBE Sequenz nur einmal vorkommenden ClaI Stelle an Position 3154. Vor dem 5'-Ende befindet sich ein weiteres Nukleotid (G), das in der nativen Sequenz des TBE-Virus nicht vorkommt, sowie eine T7 Promotor-Sequenz und die SalI Restriktionsschnittstelle, mit deren Hilfe das Fragment in den Vektor einligiert wurde. Die genaue Nukleotidsequenz des 5'-Endes und der davor liegenden Sequenzen zeigt Abb. 3. Sequenzanalysen ergeben, dass gegenüber der nativen Sequenz des TBE-Virus Stamm Neudörfl Plasmid T7-2 zwei Nukleotidänderungen aufweist (Tabelle 2):
i) eine stumme Mutation an Position 930 (T->C), die einer natürlich vorkommenden Variabilität der als Ausgangsmaterial verwendeten Viruspräparation entspricht;
ii) eine stumme Mutation an Position 3147 (C->T), die spezifisch eingeführt wurde, um die NheI Stelle an Position 3142 zu zerstören.

Die Herstellung von T7-2 erfolgte über folgende Konstruktionsschritte:

Zunächst wurde ein cDNA Fragment wie in Beispiel 1 beschrieben mittels PCR mit den Primern T01 und T45 (Tabelle 1) hergestellt und in pBR322, geschnitten mit SalI und ClaI(siehe Abb.1), einkloniert. Der erhaltene Klon enthielt die der Sequenz des TBE-Virus Stamm Neudörfl (Abb. 5) von Nukleotidposition 1 bis 3154 mit Ausnahme einer einzigen stummen Mutation an Position 930 (Tabelle 2) exakt entsprechende cDNA. Dieses Fragment wurde mit NheI (3142) und ClaI (3154) geschnitten, und das resultierende Fragment, das aus 9 Basenpaaren und zwei 4 und 2 Nukleotiden langen Überhängen (Abb. 5) besteht, durch ein mit den Oligonukleotiden T77 und T78 (Tabelle 1) gebildetes Insert ersetzt. Dadurch wurde die Mutation an Position 3147 eingeführt. Um das zusätzliche G-Nukleotid sowie die T7-Promotorsequenz am 5'-terminalen Ende der nicht-kodierenden TBEV-Sequenz einzuführen, wurde das Fragment von der 5'-terminalen, durch Primer T01 eingeführten SalI Stelle bis zur MluI Stelle (Position 208) durch ein PCR Fragment, das mit den Primern T83 (hybridisiert hinter MluI Stelle, siehe Abb. 5) und T84 (enthält eine T7-Promotorsequenz, davor eine SalI Schnittstelle und danach ein zusätzliches G gefolgt von den zwanzig 5'-terminalen Nukleotiden der TBE Sequenz; siehe Tabelle 1) hergestellt wurde, ersetzt, und Plasmid T7-2 in der oben beschriebenen Ausführung erhalten.

### Beispiel 5

### Konstruktion und Beschreibung von Plasmid 3ND-1

Plasmid 3ND-1 enthält cDNA des 3'-terminalen Bereiches **(Nukleotid 3017-11141)** des TBE-Virus Stamm Neudörfl Genoms insertiert in den Vektor pBR322. In pBR322, geschnitten mit PstI (3607) und AatII (4284), wurde ein TBEV-cDNA-Fragment, das native TBE Sequenzen von der PstI Restriktionsschnittstelle (Position 3017) bis zum 3'-Ende (Position 11141) umfasst, inseriert. Die zwei 3'-terminalen Nukleotide (CT; Abb. 3) sind Teil einer dort eingeführten NheI Stelle, gefolgt von einer AatII Stelle, mit deren Hilfe das Fragment in den Vektor einligiert wurde. Wie weiter unten ausgeführt, erlaubt die Restriktion mit NheI und die anschliessende partielle Auffüllung des sich ergebenden 5'-Überhangs die Herstellung eines der Sequenz des TBE-Virus exakt entsprechenden 3'-Endes. Die genaue Nukleotidsequenz des 3'-Endes und der benachbarten Regionen zeigt Abb. 3. Gegenüber der nativen Sequenz des TBE-Virus Stamm Neudörfl weist 3ND-1 sechs stumme Mutationen auf, die in Tabelle 2 zusammengefasst sind. Diese Mutationen wurden durch Fehler der Taq Polymerase bei der PCR verursacht.

Der Klon 3ND-1 enthält eine vollständige 3'-nicht-kodierende Sequenz, wie sie TBE-Virus Stamm Neudörfl entspricht. Eine solche Sequenz war bisher nicht bekannt. Dieser Sequenzabschnitt des Klons 3ND-1 ist in Abb.5 und 5a vollständig aufgelistet. Die Länge der internen poly(A)-Sequenz ist in nativem Virus offenbar variabel (Mandl et al., 1991), und beträgt bei Klon 3ND-1 49 Nukleotide.

Die Herstellung von Plasmid 3ND-1 erfolgte über folgende Konstruktionsschritte:

Durch direktes Klonieren von cDNA (beschrieben in Beispiel 2) wurde Klon PstI hergestellt, bei dem das von Position 3021 bis Position 6818 des TBE-Virus reichende Fragment in die singuläre PstI Stelle des Vektors pBR322 insertiert ist. Die Orientierung des Inserts war jener des Ampicillin Resistenz-Gens von pBR322 entgegengesetzt. Aus diesem Klon wurde das MluI (6446 in TBE)/ AatII (4284 in pBR322)-Fragment entfernt und durch das mit Hilfe der Primer T16 und T61 (Tabelle 1) hergestellte PCR Fragment ersetzt. Dafür wurde das 4500 Basenpaar lange PCR-Fragment mit MluI (Position 6446 der TBE Sequenz; siehe Abb. 5) und AatII (in Primer T61 enthalten) verdaut und in die entsprechenden Schnittstellen einligiert. Dadurch wurde das TBEV-spezifische Insert bis zur poly(A)-Sequenz (beginnend bei Position 10489) verlängert. cDNA, die der 3'-nicht-kodierenden Region von Stamm Neudörfl entsprach, wurde mit Hilfe des Primers H01 (Tabelle 1) hergestellt und mittels PCR mit den Primern T27 und H05 (Tabelle 1) amplifiziert und in pBR322 zwischen die Restriktionsstellen Dra I (3230) und AatII (4284) (vergleiche Abb. 1) kloniert. Aus den so erhaltenen Klonen wurde das Plasmid Dra-7, enthaltend ein Insert mit TBEV-Sequenzen von Nukleotidposition 10482 bis zum 3'-Ende (Position 11142), das eine poly(A)-Sequenz von 49 nts Länge und keine Nukleotidunterschiede zur Nativsequenz des TBE-Virus Stamm Neudörfl aufwies, ausgewählt. Das Fragment von der DraI Stelle (10484 in TBEV-Genom) bis zur AatII Schnittstelle hinter dem 3'-Ende (siehe Abb. 3) wurde an die oben beschriebene Konstruktion angefügt, um den Klon 3ND-1 in der oben beschriebenen Ausführung zu erhalten.

### Beispiel 6:

### Konstruktion und Beschreibung von Plasmid NK-4

Plasmid NK-4 enthält die vollständige cDNA Sequenz des TBE-Virus Stamm Neudörfl insertiert in den Vektor pBR322 zwischen den Restriktionsschnittstellen Sal I (651) und AatII (4284). Das 5'- und das 3'-Ende entsprechen jenen der Klone T7-2 beziehungsweise 3ND-1 (Abb. 3). Die 3'-nicht-kodierende Sequenz entspricht der in Klon 3ND-1 beschriebenen und ist in Abb. 5a dargestellt. Die insgesamt acht stummen Mutationen gegenüber der nativen Sequenz des TBE-Virus Stamm Neudörfl entsprechen jenen der oben beschriebenen Plasmide (Tabelle 2).

Zur Herstellung von Plasmid NK-4 wurde das ClaI (3154) - AatII 3'-terminale Fragment aus 3ND-1 isoliert und in Plasmid T7-2, geschnitten mit ClaI und AatII, einligiert. Das so gebildete Plasmid hatte ein Grösse von 14,9 kb und konnte nur durch Elektroporation in *E.coli* HB101 Zellen eingebracht werden. Die rekombinanten Klone vermehrten sich nur schlecht und enthielten das Plasmid NK-4 in verglichen mit pBR322 mindestens 100fach geringerer Kopienzahl.

### Beispiel 7:

### Konstruktion und Beschreibung von Plasmid 3HA

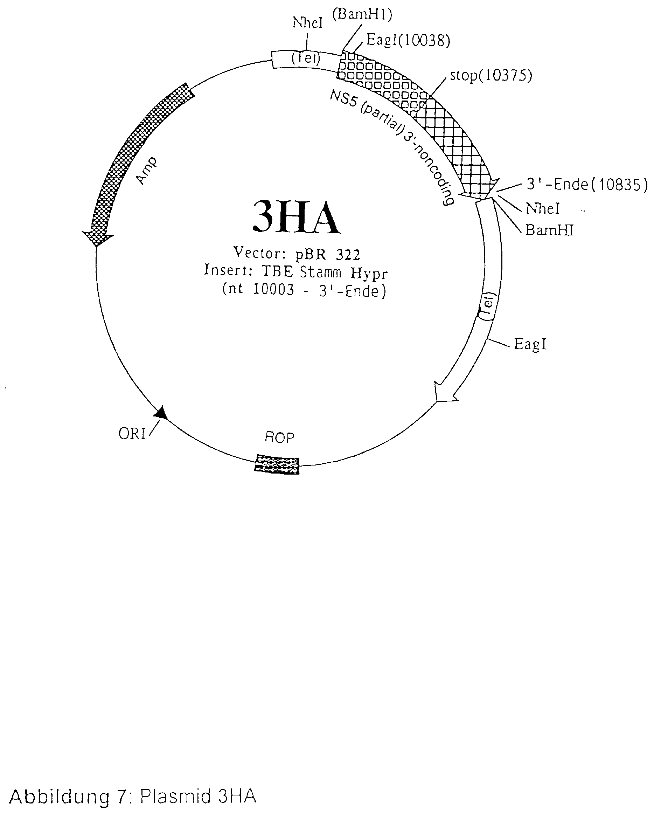

Plasmid 3HA (Abb. 7) enthält cDNA, die der 3'-terminalen Region des Genoms des TBE-Virus Stamm Hypr entspricht. Das Insert beginnt bei Nukleotid 10002 des TBE-Virus, umfasst einen Teil des für das NS5 Protein kodierenden Genomabschnittes sowie die gesamte 3'-nicht-kodierende Sequenz von TBEV Stamm Hypr bis zum 3'-Ende an Position 10835. Wie bei den Klonen 3ND-1 und NK-4 ist unmittelbar am 3'-Ende eine NheI Schnittstelle plaziert. Daran schliesst sich eine BamHI Schnittstelle, die ebenso wie eine weitere BamHI Stelle am anderen Ende des Fragments eingeführt wurde, um es in die entsprechende Restriktionsstelle des Vektors pBR322 an Position 375 einzuklonieren. Die genaue Nukleotidsequenz des 3'-Endes und der umgebenden Sequenzen zeigt Abb. 3.

Zur Herstellung von Plasmid 3HA wurde cDNA von genomischer RNA des TBE-Virus Stamm Hypr unter Verwendung des Primers H01 (Tabelle 1) hergestellt und das entsprechende Fragment mittels PCR mit Hilfe der Primer H11 und H12 (Tabelle 1) amplifiziert. Die PCR Primer bildeten an ihren 5'-Enden BamHI-Schnittstellen, die zur Klonierung des Segments in die BamHI-Stelle von pBR322 dienten. Die 5'-terminale BamHI Stelle erlitt im Zuge der Klonierung eine Deletion einer Base und liegt daher im Plasmid 3HA nicht als funktionelle Erkennungssequenz vor, wird also von BamHI nicht geschnitten. (Die entsprechende Restriktionsschnittstelle ist in Abb. 7 in Klammern angeführt.)

### Beispiel 8

### In-vitro-Transkription von RNA

Jene Plasmide, die eine T7 Promoter Sequenz enthalten (NK-4 und T7-2), beziehungsweise Ligationsprodukte dieser Plasmide mit anderen (siehe unten), wurden dazu verwendet RNA, die dem kompletten Genom des TBE-Virus entspricht, mittels in-vitro-Transkription herzustellen. Die Transkription wurde mit CsCl-Gradienten gereinigter DNA und im MEGAscrip T7 kit® (Ambion, Texas, USA) enthaltenen Reagenzien im wesentlichen entsprechend den vom Hersteller angegebenen Bedingungen durchgeführt. Die Inkubationszeit bei 37°C wurde jedoch auf 3 Std. erhöht und die an die RNA Synthese anschliessende Behandlung mit DNAseI in typischen Experimenten nicht durchgeführt.

Ausserdem wurde die Konzentration des Nukleotids GTP in jenen Ansätzen, in denen das m7G(5')ppp(5')G Cap Analog (Ambion) eingesetzt wurde, auf ein Fünftel reduziert. In ein Reaktionsvolumen von 20µl wurde circa 1µg DNA eingesetzt. Die erhaltene RNA wurde unmittelbar weiterverwendet. Die Analyse eines Aliquots solcher RNA Produkte auf Agarose-Gelen (McMaster und Carmichael, 1977) liess erkennen, dass in solchen Reaktionsansätzen von 1µg DNA ungefähr 10µg RNA, deren Grösse jener des viralen Genoms entsprach, synthetisiert wurden.

### Beispiel 9:

### Transfektion von RNA in eukaryontische Zellen und Analyse der Virusvermehrung

BHK Zellen wurden nach gängigen Laborpraktiken in 77 cm² Flaschen gezüchtet und der fast konfluente Zellrasen mit zwei 5 ml Aliquots einer Trypsin-EDTA Lösung (Gibco) abgelöst. Die Zellen wurden in 10 ml Zellkulturmedium resuspendiert und in einem 50 ml Falcon Röhrchen durch Zentrifugation in einer Sigma 4K10 Zentrifuge (800 UpM/7 min/ 20°C) pelletiert. Das Zellpellet wurde dann zweimal in kaltem (4°C) RNAse freiem PBS (Gibco) resuspendiert und abermals wie oben pelletiert. Dann wurde das Pellet in 5 ml PBS resuspendiert und in Aliquots von 0.8 ml (entsprechend circa 1 x 10⁶ Zellen) in 0.4 cm Elektroporations-Küvetten (BioRad) transferiert. RNA, die aus Viruspräparationen gereinigt worden war oder durch T7-in-vitro-Transkription hergestellt worden war, wurde direkt in die Küvetten pipettiert und vorsichtig mit der Zellsuspension vermischt. Die eingesetzten RNA Mengen lagen zwischen einigen pg RNA und einigen mg RNA. Dann wurden die Küvetten in den GenePulser der Firma BioRad eingesetzt und die Elektroporation mit folgenden Parametern durchgeführt: Spannung 1.5 kV; Kapazität 25 µF; kein Einsatz des "capacitance extenders"; Einstellung auf dem Pulse Controller auf "infinity". Unter diesen Bedingungen wurde als Zeitkonstante üblicherweise 0.9 erhalten. 10 sec nach dem ersten Stromstoss wurde ein zweiter ausgeführt und dann die Küvette für 10 min bei Zimmertemperatur stehen gelassen. Erst dann wurden die Zellen in eine neue Zellkulturflasche gesät und für die nächsten Tage in Medium, das 5% fötales Kälberserum enthielt, gezüchtet. Sobald die Zellen konfluent waren, wurde die Serumkonzentration auf 1% abgesenkt.

Zur Überprüfung der Virusvermehrung wurden täglich 50µl Aliquote der Zellkulturflüssigkeit entnommen und bei -20°C tiefgefroren. Mit Hilfe eines 4-Schicht ELISA (Heinz et al. 1986) wurde das TBE-Virus in den Zellkulturüberständen nachgewiesen.

### Beispiel 10:

### Herstellung von TBE-Virus Stamm Neudörfl von rekombinanter cDNA

TBE-Virus Stamm Neudörfl konnte auf zwei verschiedene Arten von den beschriebenen rekombinanten Plasmiden generiert werden:
i) 1 µg NK-4 wurde mit 5 E des Restriktionsenzyms NheI linearisiert und durch Phenol/Chloroformextraktion gereinigt und mit Ethanol gefällt. Die DNA wurde dann in Klenow Reaktionspuffer (New England Biolabs) gelöst und in Gegenwart der Nukleotide dCTP und dTTP (Boehringer-Mannheim) für 15 min bei 37°C mit dem Enzym Klenow (New England Biolabs) in einem Reaktionsvolumen von insgesamt 30 µl inkubiert. Das Reaktionsprodukt wurde neuerlich wie oben gereinigt und dann in die RNA Transkriptionsreaktion eingesetzt.
ii) Mehrere µg 3ND-1 wurden wie oben beschrieben mit NheI geschnitten und der 5'-Überhang durch die Klenow-Reaktion teilweise aufgefüllt. Dann wurde das Plasmid mit ClaI geschnitten und das ungefähr 8000 nts lange TBE-spezifische Fragment auf 0.3% SeaKem Gold Agarose (FMC) von den anderen Reaktionsprodukten abgetrennt und durch Elekroelution gereinigt. Danach wurde ein Aliquot dieser Präparation zur Quantifizierung auf einem Agarose-Gel analysiert. 500 ng T7-2 wurden mit ClaI linearisiert und mit ungefähr 1-2 µg des gereinigten 3ND-1 Fragments vereinigt. Das Gemisch wurde gereinigt und in 30 µl Volumen mit T4 DNA Ligase (Stratagene) über Nacht bei 16°C ligiert. Der Ligationsansatz wurde dann bei 65°C 5 min inaktiviert und neuerlich gereinigt und für die in vitro-RNA-Transkriptionsreaktion eingesetzt.

RNA, die nach einer dieser beiden Methoden hergestellt worden war, wurde in BHK Zellen transformiert (Elektroporation) und die Virusvermehrung durch 4-Schicht ELISA des Zellkulturüberstandes beobachtet. Die Antigenstruktur des so gebildeten rekombinanten Virus wurde mit Hilfe einer Anzahl monoklonaler Antikörper, die gegen das Hüllprotein des TBE-Virus gerichtet sind (Heinz et al., 1983; Guirakhoo et al., 1989), analysiert und entsprach völlig dem des nativen TBE-Virus Stamm Neudörfl.

Die spezifische Infektiosität der nach dieser Methode aus NK-4 hergestellten RNA wurde mit jener von aus Viruspräparationen gereinigter RNA verglichen. Sie betrug für beide RNA Präparationen in verschiedenen Experimenten zwischen 5 und 50 pg RNA pro infektiöser Einheit (bei Elektroporation auf BHK Zellen wie beschrieben). Die Behandlung der Transkriptionsreaktion mit DNAseI nach der Transkription hatte keinen Einfluss auf die spezifische Infektiosität der RNA. RNA, die ohne CAP-Analog hergestellt wurde, wies eine 1000-fach geringere spezifische Infektiosität auf. Wurde das Plasmid nicht mit NheI und anschliessender Klenow-Inkubation behandelt, sondern mit AatII geschnitten, so ergab sich eine ungefähr 10-fach reduzierte spezifische Infektiosität. Allerdings bleibt dabei unklar, ob das 3'-Ende des Genoms des rekombinanten Virus mit jenem des nativen Virus identisch oder ob es zusätzliche Nukleotide enthält, da die AatII Stelle einige Nukleotide nach der NheI Stelle liegt (siehe Abb. 3).

### Beispiel 11:

### Herstellung von TBE-Virus Stamm Neudörfl, welches das 3'-Ende eines anderen Stammes (Stamm Hypr) enthält

Mehrere µg 3HA wurden mit Nhe I geschnitten (ergibt 2 Fragmente: 4210bp, 980 bp) und mit Klenow-Enzym inkubiert, wie oben für NK-4 beschrieben. Das Plasmid wurde mit Eag I weiter verdaut (ergibt 4 Fragmente: 3650; 800; 560; 180) und das entstandene 800 Nukleotide lange TBEV-spezifische Fragment aus einem 1% Agarose-Gel gereinigt. 1 µg NK-4 wurde ebenfalls mit EagI geschnitten und mit ungefähr 200 ng des gereinigten NheI-EagI-Fragments von Plasmid HA3 vereinigt. Das Gemisch wurde gereinigt, ligiert und transkribiert und die RNA, wie oben beschrieben, in BHK Zellen transformiert. Aus dem gebildeten rekombinanten Virus wurde RNA isoliert und in cDNA überführt. Von dieser cDNA wurden durch PCR einige kurze Abschnitte amplifiziert und die Sequenz dieser Fragmente direkt ohne vorherige Klonierung analysiert. Diese Sequenzanalysen bestätigten die erwartete Genomstruktur des rekombinanten Virus, mit der Sequenz des TBE-Virus Stamm Neudörfl bis zur Position 10038 und jener des TBE-Virus Stamm Hypr von 10039 bis zum 3'-Ende. Die mit monoklonalen Antikörpern (Guirakhoo et al. 1989; Holzmann et al. 1992) bestimmte Antigenstruktur entsprach vollkommen jener des Stammes Neudörfl, jedoch zeigte das rekombinante Virus in BHK Zellen eher den für Stamm Hypr charakteristischen stärkeren zytopathischen Effekt als jenen schwächeren, den das native TBE-Virus Stamm Neudörfl hervorruft.

### Beispiel 12:

### Herstellung einer attenuierten Mutante des TBE-Virus unter Verwendung von rekombinanter cDNA

Eine Punktmutation des TBE-Virus Stamm Neudörfl, die zu einer Änderung der Aminosäuresequenz des E Proteins an Position 384 von Tyrosin zu Histidin führt, hat eine signifikante Attenuierung des Virus in Bezug auf seine Neurovirulenz und seine Neuroinvasivität im Tiermodell (Maus) zur Folge (Holzmann et al.,1990). Diese attenuierte Mutante des TBE-Virus wurde nun auch mit Hilfe des erfindungsgemässen infektiösen Klons hergestellt:

Das Plasmid T7-2 wurde mit den Enzymen BamHI (1988) und ClaI (3154) geschnitten, die beiden entstehenden Fragmente der Länge von 5740 bp und 1170 bp wurden auf einem 1% Agarose-Gel aufgetrennt und eluiert. Das 1170 bp lange Fragment wurde mit HinfI (Schnittstelle in TBEV an Position 2136) geschnitten und die resultierenden Fragmente der Länge 1020 bp und 150 bp aufgetrennt und das 1020 bp lange Fragment eluiert. Ungefähr gleiche Mengen des erhaltenen BamHI/ClaI-Fragments (5740 bp) und HinfI/ClaI-Fragments (1020 bp) wurden nun mit einem 4- 5-fachen molaren Überschuss eines kleinen PCR-Fragments gemischt, das die gewünschte Mutation enthielt und wie folgt gewonnen wurde: 1 ng Plasmid T7-2 wurde als Ausgangsmaterial in eine PCR Reaktion mit den Primern T91 und Mut01 (Tabelle 1) eingesetzt. Der Primer Mut01 umspannt sowohl die oben erwähnte HinfI-Stelle (2136) als auch die zu mutierende Nukleotidposition (G statt A am Minusstrang des Genoms an Position 2103). Primer T91 wurde so positioniert, dass das entstehende PCR-Fragment auch die BamHI-Stelle bei Nukleotidposition 1988 enthält. Das erhaltene PCR-Produkt wurde mit den Enzymen BamHI und HinfI geschnitten, über ein 1.2% Agarose-Gel getrennt und das 150 bp lange BamHI/HinfI-Fragment durch Elektroelution isoliert und, wie oben angegeben, mit den beiden anderen Fragmenten gemischt. Das Gemisch dieser drei Fragmente wurde gereinigt, ligiert und in *E.coli* HB101 transformiert. Rekombinante Plasmide wurden isoliert und die gewünschte Sequenz durch Sequenzanalyse bestätigt. Das so hergestellte Plasmid, das sich nur in einer einzigen Punktmutation von dem Plasmid T7-2 unterschied, konnte in gleicher Weise wie T7-2 zur Herstellung von TBE-Virus verwendet werden (siehe oben). Das erhaltene rekombinante Virus (RB4) wurde mit den monoklonalen Antikörpern ( Heinz et al. 1983; Guirakhoo et al. 1989) analysiert, und wies exakt dasselbe Reaktionsmuster auf wie die von Holzmann et al. (1990) beschriebene attenuierte B4-Mutante.

### Beispiel 13:

### Charakterisierung der Virulenzeigenschaften der rekombinanten Mutante RB4

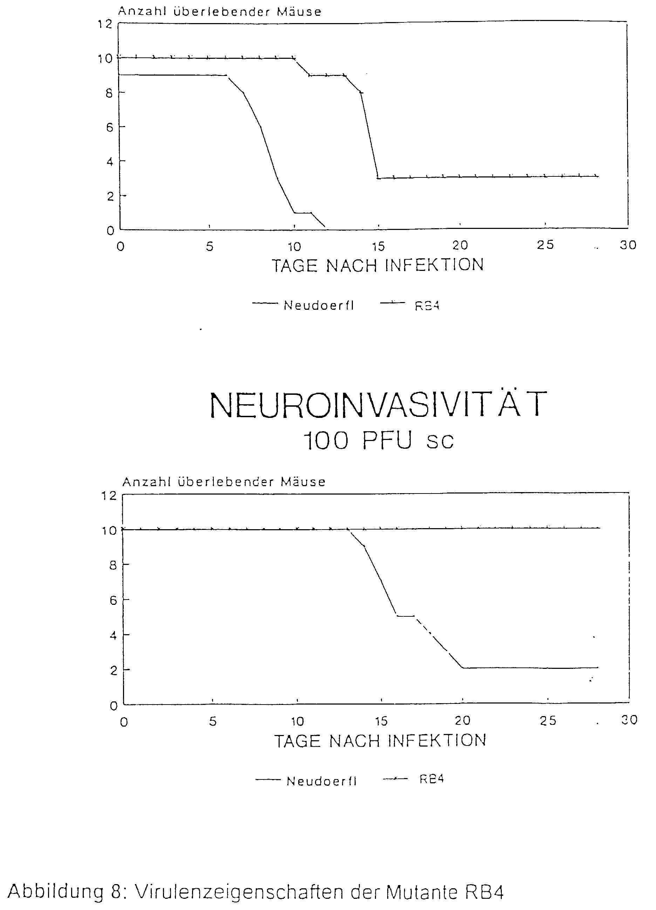

Zur Virulenzbestimmung wurden erwachsene Swiss Albino Mäuse verwendet, die sowohl nach intrazerebraler als auch nach subcutaner Infektion mit dem Wildtyp Stamm Neudörfl eine tödlich verlaufende Enzephalitis entwickeln. Gruppen von je 10 Mäusen wurden mit gleichen Mengen, nämlich jeweils 100 PFU (Plaque Forming Units), des Stammes Neudörfl und der Mutante RB4 intrazerebral bzw. subcutan infiziert. Wie aus der Abb. 8 hervorgeht, war sowohl die Neurovirulenz (i.c. Inokulation), als auch die Neuroinvasivität (s.c. Inokulation) der Mutante RB4 im Vergleich zum Stamm Neudörfl stark reduziert. Das mit Hilfe des infektiösen Klons hergestellte Virus RB4 entspricht in seinen Virulenzeigenschaften somit der mit Hilfe eines monoklonalen Antikörpers selektionierten Variante VB4 (Holzmann et al., 1990).

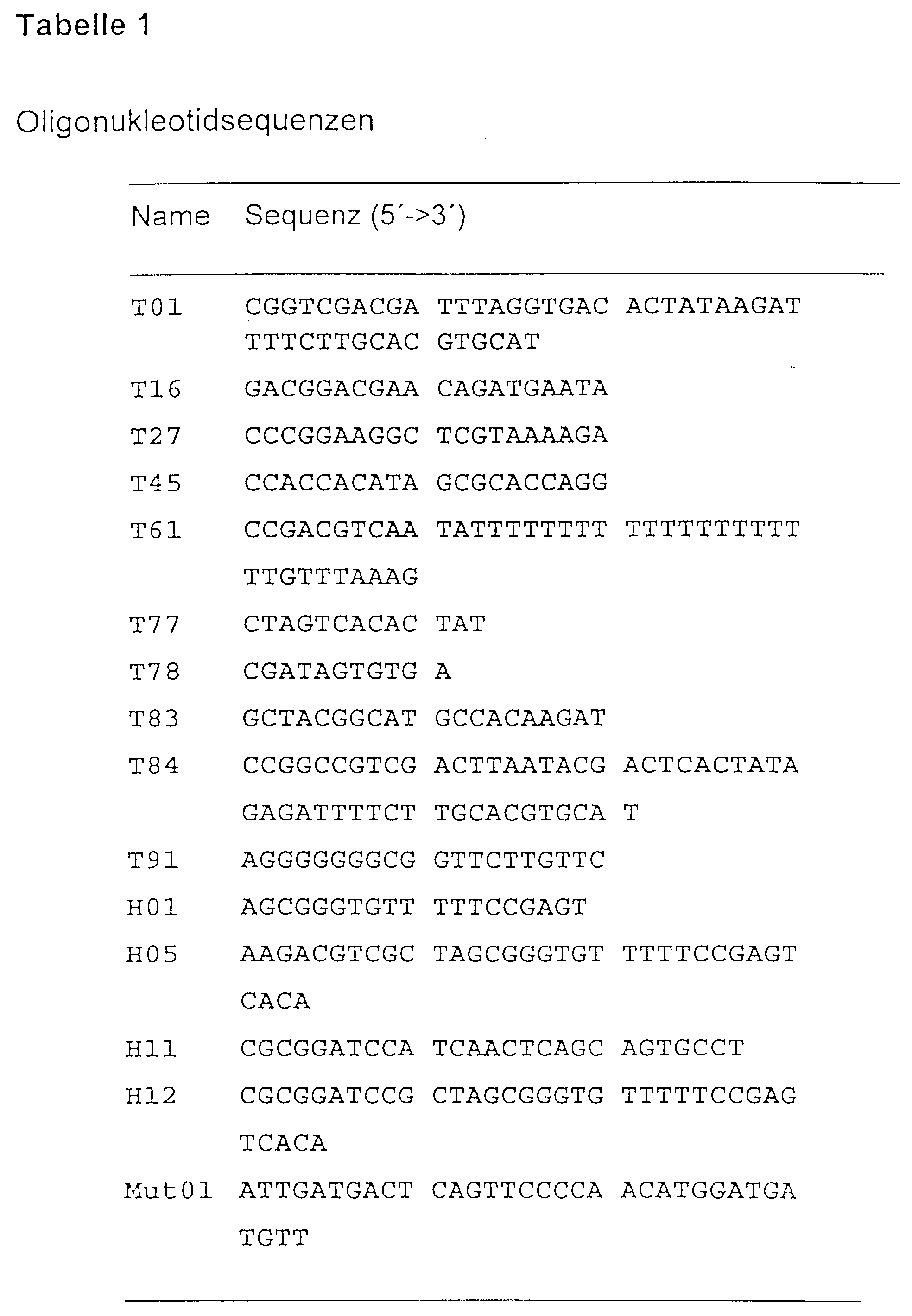

**Tabelle 2**

| Sequenzunterschiede zwischen infektiösem Klon und der Wildtypsequenz von TBE Stamm Neudörfl | | |
|---|---|---|
| Position | Unterschied (Neudörfl->I.K.) | Anmerkung |
| "0" | G | zusätzliches Nukleotid am 5'-Ende; in T7-2, NK-4 |
| 930 | T->C | in T7-2, NK-4 |
| 3147 | C->T | Nhe I Stelle spezifisch entfernt; in T7-2, NK-4 |
| 4860 | C->T | in 3ND-1, NK-4 |
| 7434 | G->A | in 3ND-1, NK-4 |
| 7464 | G->T | in 3ND-1, NK-4 |
| 7617 | T->C | in 3ND-1, NK-4 |
| 7638 | C->A | in 3ND-1, NK-4 |
| 8493 | A->G | in 3ND-1, NK-4 |

**Tabelle 3**

| **Sequenzunterschiede zwischen TBE Virus Prototyp Stamm Neudörfl und dem attenuierten natürlichen Isolat 263** | | |
|---|---|---|
| Position^{a} | Nukleotid^{b} | Aminosäure^{c} |
| **5' - NC** | | |
| 49 | G - T | |

| **C** | | |
|---|---|---|
| 183 | A - C | |
| 285 | C - T | |
| 307 | T - C | |
| 345 | A - C | |
| 375 | G - A | |
| 432 | C - T | |
| 456 | C - T | |

| **prM** | | |
|---|---|---|
| 504 | T - C | |
| 585 | T - C | |
| 591 | C - T | |
| 697 | T - C | Tyr - His (73) |
| 708 | C - T | |

| **M** | | |
|---|---|---|
| 754 | C - T | |
| 789 | A - G | |
| 798 | C - T | |
| 834 | T - C | |
| 846 | G - A | |
| 882 | A - G | |
| 922 | A - G | Thr - Ala (60) |
| 930 | T - C | |
| 939 | T - G | |
| 960 | A - G | |
| 963 | G - T | |
| 966 | T - C | |

| **E** | | |
|---|---|---|
| 1014 | T - C | |
| 1035 | C - T | |
| 1065 | T - C | |
| 1068 | T - C | |
| 1127 | A - G | Asn - Ser (52) |
| 1173 | C - T | |
| 1191 | A - G | |
| 1200 | A - G | |
| 1471 | A - G | Ile - Val (167) |
| 1492 | T - C | |
| 1497 | T - C | |
| 1620 | T - C | |
| 1680 | C - T | |
| 1833 | C - T | |
| 1932 | C - T | |
| 1963 | A - T | Thr - Ser (331) |
| 2037 | C - T | |
| 2160 | G - A | |
| 2283 | A - G | |
| 2286 | T - C | |
| 2301 | C - T | |
| 2328 | G - A | |
| 2343 | A - G | |
| 2412 | T - C | |
| 2439 | C - T | |

| **NS1** | | |
|---|---|---|
| 2469 | T - C | |
| 2541 | A - G | |
| 2553 | T - C | |
| 2613 | G - A | |
| 2628 | T - C | |
| 2658 | C - A | |
| 2689 | T - C | |
| 2859 | C - T | |
| 3015 | A - G | |
| 3021 | A - C | |
| 3068 | G - A | Arg - Lys (203) |
| 3180 | A - G | |
| 3185 | T - C | Leu - Ser (242) |
| 3225 | C - T | |
| 3237 | T - C | |
| 3240 | C - T | |
| 3271 | T - C | Tyr - His (271) |
| 3288 | T - C | |
| 3309 | C - A | |
| 3363 | T - C | |
| 3486 | G - A | |

| **NS2A** | | |
|---|---|---|
| 3529 | T - C | |
| 3546 | A - G | |
| 3600 | G - A | |
| 3615 | G - A | |
| 3633 | T - C | |
| 3634 | A - T | Ile - Phe (40) |
| 3642 | T - A | |
| 3711 | C - T | |
| 3729 | G - A | |
| 3732 | G - A | |
| 3759 | C - T | |
| 3805 | T - C | |
| 3897 | G - T | Glu - Asp (127) |
| 3898 | T - C | Phe - Leu (128) |
| 3936 | G - A | |
| 3981 | T - C | |
| 4026 | C - T | |
| 4047 | A - G | |
| 4062 | A - T | |
| 4117 | G - A | Val - Ile (201) |
| 4132 | G - A | |
| 4134 | G - A | Gly - Arg (206) |
| 4135 | T - C | |
| 4164 | A - G | |
| 4183 | T - C | |
| 4186 | T - G | Ser - Ala (224) |
| 4191 | G - T | |

| **NS2B** | | |
|---|---|---|
| 4276 | T - C | Ser - Pro (24) |
| 4380 | T - G | |
| 4387 | T - C | Tyr - His (61) |
| 4434 | G - T | |
| 4449 | T - A | |
| 4504 | A - C | Ile - Leu (100) |
| 4506 | T - G | |
| 4533 | C - T | |

| **NS3** | | |
|---|---|---|
| 4611 | T - C | |
| 4644 | C - T | |
| 4683 | T - C | |
| 4713 | G - A | |
| 4737 | G - A | |
| 4794 | C - T | |
| 4816 | G - T | Ala - Ser (73) |
| 4842 | T - C | |
| 4916 | G - A | Arg - Lys (106) |
| 4920 | G - C | |
| 4971 | A - G | |
| 5001 | G - A | |
| 5058 | C - T | |
| 5106 | A - G | |
| 5127 | C - A | |
| 5154 | T - C | |
| 5298 | A - G | |
| 5316 | T - C | |
| 5319 | T - C | |
| 5381 | T - C | |
| 5422 | A - C | |
| 5439 | A - G | |
| 5496 | C - T | |
| 5520 | T - C | |
| 5548 | C - T | |
| 5553 | C - T | |
| 5604 | T - C | |
| 5643 | G - A | |
| 5679 | T - C | |
| 5740 | T - C | |
| 5766 | T - C | |
| 5862 | T - C | |
| 5910 | C - T | |
| 5928 | A - G | |
| 5964 | T - G | |
| 6000 | A - G | |
| 6015 | T - C | |
| 6102 | A - G | |
| 6106 | T - C | |
| 6267 | A - G | |
| 6345 | C - T | |
| 6381 | C - T | |
| 6405 | T - C | |

| **NS4A** | | |
|---|---|---|
| 6483 | A - G | |
| 6501 | T - G | |
| 6507 | C - T | |
| 6508 | C - T | |
| 6537 | T - C | |
| 6549 | G - A | |
| 6552 | T - C | |
| 6655 | C - T | |
| 6732 | C - G | |
| 6738 | A - G | |
| 6777 | T - C | |
| 6795 | T - C | |
| 6822 | T - C | |

| **NS4B** | | |
|---|---|---|
| 6915 | G - A | |
| 6921 | C - T | |
| 6956 | C - T | Ala - Val (16) |
| 6958 | C - T | |
| 6975 | A - G | Glu - Gly (22) |
| 7014 | C - T | |
| 7062 | C - T | |
| 7110 | C - T | |
| 7224 | T - C | |
| 7233 | C - T | |
| 7254 | C - T | |
| 7320 | T - C | |
| 7442 | C - T | Thr - Ile (178) |
| 7458 | G - T | Met - Ile (183) |
| 7506 | A - T | |
| 7539 | A - C | Arg - Ser (210) |
| 7554 | G - A | |
| 7555 | T - C | |
| 7618 | T - C | |
| 7650 | C - T | |
| 7659 | T - C | |

| **NS5** | | |
|---|---|---|
| 7704 | G - A | |
| 7725 | G - C | |
| 7740 | T - C | |
| 7755 | C - T | |
| 7803 | A - G | |
| 7816 | G - A | Val - Met (51) |
| 7974 | T - C | |
| 7988 | A - G | Lys - Arg (108) |
| 8058 | C - T | |
| 8161 | C - T | |
| 8184 | C - T | |
| 8247 | G - A | |
| 8269 | C - A | Gln - Lys (202) |
| 8340 | C - T | |
| 8361 | C - T | |
| 8370 | T - C | |
| 8371 | G - A | Val - Ile (263) |
| 8423 | A - G | Lys - Arg (253) |
| 8436 | C - T | |
| 8454 | G - T | |
| 8455 | A - C | |
| 8469 | A - G | |
| 8508 | G - A | |
| 8529 | A - G | |
| 8547 | T - C | |
| 8694 | T - C | |
| 8736 | A - G | |
| 8760 | G - A | |
| 8796 | T - A | |
| 8805 | T - C | |
| 8841 | G - A | |
| 8855 | G - A | Arg - Lys (397) |
| 8856 | G - A | |
| 8859 | A - G | |
| 8966 | G - A | Arg - His (434) |
| 9009 | T - C | |
| 9022 | T - C | Tyr - His (453) |
| 9045 | A - G | |
| 9052 | C - T | |
| 9060 | G - A | |
| 9118 | T - C | |
| 9306 | C - T | |
| 9307 | T - C | |
| 9348 | G - A | |
| 9358 | T - C | |
| 9417 | T - C | |
| 9462 | C - T | |
| 9471 | T - C | |
| 9477 | T - C | |
| 9498 | T - C | |
| 9562 | T - C | |
| 9759 | A - G | |
| 9786 | A - C | |
| 9828 | T - C | |
| 9930 | T - C | |
| 9972 | C - T | |
| 9975 | A - G | |
| 10005 | T - C | |
| 10022 | C - T | Ala - Val (786) |
| 10050 | G - A | |
| 10059 | T - C | |
| 10104 | T - C | |
| 10123 | C - T | |
| 10154 | G - A | Arg - Lys (830) |
| 10171 | G - A | Asp - Asn (836) |
| 10194 | T - C | |
| 10229 | G - A | Arg - Lys (855) |
| 10236 | A - G | |

| **3' - NC** | | |
|---|---|---|
| 10407 | A - G | |
| 10438 | A - G | |
| 10452 | C - T | |

| | | |
|---|---|---|
| ^{a} Position in der Nukleotidsequenz von TBE Virus Stamm Neudörfl. | | |
| ^{b} Das linke Nukleotid entspricht der Sequenz von Stamm Neudörfl, das rechte jener von Stamm 263 | | |
| ^{c} Die linke Aminosäure entspricht der Sequenz von Stamm Neudörfl, die rechte jener von Stamm 263. Die Zahl in Klammern bezieht sich auf die Aminosäureposition gezählt vom Aminoterminus des jeweiligen Proteins. | | |

**Tabelle 3a**

| **Beispielhafte Modifikationen der Nukleotidsequenz des TBE - Virus Stamm Neudörfl zur Attenuiereung des Virus** | | | |
|---|---|---|---|
| Position^{a} | Nukleotid^{b} | Position^{a} | Nukleotid^{b} |
| | | 1471 | A - G |
| **5' - NC** | | 1492 | T - C |
| 49 | G - T | 1497 | T - C |
| **C** | | 1620 | T - C |
| 183 | A - C | 1680 | C - T |
| 285 | C - T | 1833 | C - T |
| 307 | T - C | 1932 | C - T |
| 345 | A - C | 1963 | A - T |
| 375 | G - A | 2037 | C - T |
| 432 | C - T | 2160 | G - A |
| 456 | C - T | 2283 | A - G |
| **prM** | | 2286 | T - C |
| 504 | T - C | 2301 | C - T |
| 585 | T - C | 2328 | G - A |
| 591 | C - T | 2343 | A - G |
| 697 | T - C | 2412 | T - C |
| 708 | C - T | 2439 | C - T |
| **M** | | **NS1** | |
| 754 | C - T | 2469 | T - C |
| 789 | A - G | 2541 | A - G |
| 798 | C - T | 2553 | T - C |
| 834 | T - C | 2613 | G - A |
| 846 | G - A | 2628 | T - C |
| 882 | A - G | 2658 | C - A |
| 922 | A - G | 2689 | T - C |
| 930 | T - C | 2859 | C - T |
| 939 | T - G | 3015 | A - G |
| 960 | A - G | 3021 | A - C |
| 963 | G - T | 3068 | G - A |
| 966 | T - C | 3180 | A - G |
| **E** | | 3185 | T - C |
| 1014 | T - C | 3225 | C - T |
| 1035 | C - T | 3237 | T - C |
| 1065 | T - C | 3240 | C - T |
| 1068 | T - C | 3271 | T - C |
| 1127 | A - G | 3288 | T - C |
| 1173 | C - T | 3309 | C - A |
| 1191 | A - G | 3363 | T - C |
| 1200 | A - G | 3485 | G - A |
| **NS2A** | | | |
| 3529 | T - C | 4916 | G - A |
| 3546 | A - G | 4920 | G - C |
| 3600 | G - A | 4971 | A - G |
| 3615 | G - A | 5001 | G - A |
| 3633 | T - C | 5058 | C - T |
| 3634 | A - T | 5106 | A - G |
| 3642 | T - A | 5127 | C - A |
| 3711 | C - T | 5154 | T - C |
| 3729 | G - A | 5298 | A - G |
| 3732 | G - A | 5316 | T - C |
| 3759 | C - T | 5319 | T - C |
| 3805 | T - C | 5381 | T - C |
| 3897 | G - T | 5422 | A - C |
| 3898 | T - C | 5439 | A - G |
| 3936 | G - A | 5496 | C - T |
| 3981 | T - C | 5520 | T - C |
| 4026 | C - T | 5548 | C - T |
| 4047 | A - G | 5553 | C - T |
| 4062 | A - T | 5604 | T - C |
| 4117 | G - A | 5643 | G - A |
| 4132 | G - A | 5679 | T - C |
| 4134 | G - A | 5740 | T - C |
| 4135 | T - C | 5766 | T - C |
| 4164 | A - G | 5862 | T - C |
| 4183 | T - C | 5910 | C - T |
| 4186 | T - G | 5928 | A - G |
| 4191 | G - T | 5964 | T - G |
| **NS2B** | | 6000 | A - G |
| 4276 | T - C | 6015 | T - G |
| 4380 | T - G | 6102 | A - G |
| 4387 | T - C | 6106 | T - C |
| 4434 | G - T | 6267 | A - G |
| 4449 | T - A | 6345 | C - T |
| 4504 | A - C | 6381 | C - T |
| 4506 | T - G | 6405 | T - C |
| 4533 | C - T | **NS4A** | |
| **NS3** | | 6483 | A - G |
| 4611 | T - C | 6501 | T - G |
| 4644 | C - T | 6507 | C - T |
| 4683 | T - C | 6508 | C - T |
| 4713 | G - A | 6537 | T - C |
| 4737 | G - A | 6549 | G - A |
| 4794 | C - T | 6552 | T - C |
| 4816 | G - T | 6655 | C - T |
| 4842 | T - C | 6732 | C - G |
| 6738 | A - G | 8455 | A - C |
| 6777 | T - C | 8469 | A - G |
| 6795 | T - C | 8508 | G - A |
| 6822 | T - C | 8529 | A - G |
| **NS4B** | | 8547 | T - C |
| 6915 | G - A | 8694 | T - C |
| 6921 | C - T | 8736 | A - G |
| 6956 | C - T | 8760 | G - A |
| 6958 | C - T | 8796 | T - A |
| 6975 | A - G | 8805 | T - C |
| 7014 | C - T | 8841 | G - A |
| 7062 | C - T | 8855 | G - A |
| 7110 | C - T | 8856 | G - A |
| 7224 | T - C | 8859 | A - G |
| 7233 | C - T | 8966 | G - A |
| 7254 | C - T | 9009 | T - C |
| 7320 | T - C | 9022 | T - C |
| 7442 | C - T | 9045 | A - G |
| 7458 | G - T | 9052 | C - T |
| 7506 | A - T | 9060 | G - A |
| 7539 | A - C | 9118 | T - C |
| 7554 | G - A | 9306 | C - T |
| 7555 | T - C | 9307 | T - C |
| 7618 | T - C | 9348 | G - A |
| 7650 | C - T | 9358 | T - C |
| 7659 | T - C | 9417 | T - C |
| **NS5** | | 9462 | C - T |
| 7704 | G - A | 9471 | T - C |
| 7725 | G - C | 9477 | T - C |
| 7740 | T - C | 9498 | T - C |
| 7755 | C - T | 9562 | T - C |
| 7803 | A - G | 9759 | A - G |
| 7816 | G - A | 9786 | A - C |
| 7974 | T - C | 9828 | T - C |
| 7988 | A - G | 9930 | T - C |
| 8058 | C - T | 9972 | C - T |
| 8161 | C - T | 9975 | A - G |
| 8184 | C - T | 10005 | T - C |
| 8247 | G - A | 10022 | C - T |
| 8269 | C - A | 10050 | G - A |
| 8340 | C - T | 10059 | T - C |
| 8361 | C - T | 10104 | T - C |
| 8370 | T - C | 10123 | C - T |
| 8371 | G - A | 10154 | G - A |
| 8423 | A - G | 10171 | G - A |
| 8436 | C - T | 10194 | T - C |
| 8454 | G - T | 10229 | G - A |
| **3' - NC** | | | |
| 10407 | A - G | | |
| 10438 | A - G | | |
| 10452 | C - T | | |

| | | | |
|---|---|---|---|
| ^{a} Position in der Nukleotidsequenz von TBE Virus Stamm Neudörfl. | | | |
| ^{b} Das linke Nukleotid entspricht der Sequenz von Stamm Neudörfl, das rechte der eingeführten Modifikation. | | | |

### Literaturverzeichnis

CHAMBERS, T.J., HAHN, C.S., GALLER, R., RICE, C.M. (1990). Flavirus genome organization, expression and replication. Ann. rev. Microbiol. 44:649-688.

EMINI, E.A., SCHLEIF, W.A., NUNBERG, J.H., CONLEY, A.J., EDA, Y., TOKIYOSHI, S., PUTNEY, S.D., MATSUSHITA, S., COBB, K.E., JETT, C.M., EICHBERG, J.W., AND MURTHY, K.K. (1992). Prevention of HIV-1 infection in chimpanzees by gp 120 V3 domain-specific monoclonal antibody. *Nature* **355**, 728-729.

Gao, G.F., Jiang, W.R., Hussain, M.H., Venugopal, K., Gritsun, T.S., Reid, H.W., and Gould, E.A. (1993). Sequencing and antigenic studies of a Norwegian virus isolated from encephalomyelitic sheep confirm the existence of louping ill outside Great Britain and Ireland. J. Gen. Virol. **74**, 109-114

GUBLER, U., AND HOFFMAN, B.J. (1983). A simple and very efficient method for generating cDNA libraries. *Gene* **25**, 263-269.

GUIRAKHOO, F., HEINZ, F.X., and KUNZ, C. (1989). Epitope model of tick-borne Encephalitis virus envelope glycoprotein E: Analysis of structural properties, role of carbohydrate side chain, and conformational changes occuring at acidic pH. *Virology* **169**, 90-99.

HASLOV, K., PETERSON, J.W., BAKER, P.J. (1991). Adjuvanticity of pertussis toxin is mediated by differential effects on the activity of T suppresors, T amplifier and T helper cell. Immunobiol. 183:40-54

HAZAME M., MAYUMI-AONO, A., ASAKAWA, N., KURODA, S., HINUMA, S., AND FUJISAWA, Y. (1993). Adjuvant-independent enhanced immune responses to recombinant herpes simplex virus type 1 glycoprotein D by fusion with biologically active interleukin-2. *Vaccine* **11**, 629-635.

HEINZ, F.X., BERGER, R., TUMA, W., and KUNZ, C. (1983). A topological and functional model of epitopes on the structural glycoprotein of tick-borne encephalitis virus defined by monoclonal antibodies. *Virology* **126**, 525-537.

HEINZ, F.X., AND KUNZ C. (1981). Homogeneity of the structural glycoprotein from European isolates of tick-borne encephalitis virus: Comparison with other flaviviruses. *J. Gen. Virol*. **57**, 263-274.

HEINZ, F.X., TUMA W., GUIRAKHOO, F., AND KUNZ, C. (1986). A model study of the use of monoclonal antibodies in capture enzyme immunoassays for antigen quantification exploiting the epitope map of tick-borne encephalitis virus. *J.Biol.Standard*. **14**, 133-141.

HOLZMANN, H., HEINZ, F.X., MANDL, C.W., GUIRAKHOO, F., AND KUNZ, C. (1990). A single amino acid substitution in the envelope protein E of tick-borne encephalitis virus leads to attenuation in the mouse model. *J. Virol*. **64**, 5156-5159.

Holzmann, H., Vorobyova, M.S., Ladyzhenskaya, I.P., Ferenczi, E., Kundi, M., Kunz, C., and Heinz, F.X. (1992). Molecular epidemiology of tick-borne encephalitis virus: cross-protection between European and Far Eastern subtypes. Vaccines **10**, 345-349

HUSSAIN, A., HIMENO, K.,MAYUMI, H., KAWAMURA, I., TSURU, S., NOMOTO, K. (1989). Immunomodulatory effects of cholera toxin in mice. Nat. Immun. Cell. Growth Regul. 8:231-244.

JAVAHERIAN, K., LANGLOIS, A.J., MCDANAL, C., Ross, K.L., ECKLER, L.I., JELLIS, C.L., PROFY, A.T., RUSCHE, J.R., BOLOGNESI, D.P., PUTNEY, S.D., AND MATTHEWS, T.J. (1989). Principal neutralizing domain of the human immunodeficiency virus type 1 envelope protein. *Proc.Natl.Acad.Sci. USA* **86**, 6768-6772.

LAI, C., IHAO, B., HORI, H., BRAY, M. (1991). Infectious RNA transcribed from cloned full-lenght cDNA of Dengue type 4 virus. Proc. Natl. Acad. Sci. USA. 88:5139-5143.

Ledger, T.N., Sil, B.K., Wills, M.R., Lewis, G., Kinney, R.M., Jennings, J.R., Stephenson, J.R., and Barrett, A.D.T. (1992). Variation in the biological function of envelope protein epitopes of yellow fever vaccine viruses detected with monoclonal antibodies. Biologicals **20**, 117-128

MANDL, C.W., HEINZ, F.X., AND KUNZ, C. (1988). Sequence of the structural proteins of tick-borne encephalitis virus (Western subtype) and comparative analysis with other flaviviruses. *Virology* **166**, 197-205.

MANDL, C.W., HEINZ, F.X., STÖCKL, E., AND KUNZ, C. (1989). Genome sequence of tick-borne encephalitis virus (Western subtype) and comparative analysis of nonstructural proteins with other flaviviruses. *Virology* **173**, 291-301.

MANDL, C.W., HOLZMANN, H., KUNZ, C., AND HEINZ, F.X. (1993). Complete genomic sequence of Powassan virus: Evaluation of genetic elements in tick-borne versus mosquito-borne flaviviruses. *Virology* **194**, 173-184.

MANDL, C.W., KUNZ, C., AND HEINZ, F.X. (1991). Presence of poly(A) in a flavivirus: Significant differences between the 3' noncoding regions of the genomic RNAs of tick-borne encephalitis virus strains. *J. Virol*. **65**, 4070-4077.

MANIATIS, T., FRITSCH, E.F., AND SAMBROOK, J. (eds.) (1982). "Molecular Cloning, a Laboratory Manual", Cold Spring Harbor Laboratory, Cold Spring Harbor, New York.

MCMASTER, G.K., AND CARMICHAEL, G.G. (1977). Analysis of single- and double-stranded nucleic acids on polyacrylamide and agarose gels by using glyoxal and acridine orange. *Proc.Natl.Acad.Sci. USA* **11**, 4835-4838.

Monath, T.P. (1990), Flaviviruses In: Fidds, B.N. (ed) Virology, Roven Press, pp. 107-151.

Pletnev, A.G., Bray, M., and Lai, C.-J. (1993).Chimeric tick-borne encephalitis and dengue type 4 viruses: Effects of mutations on neurovirulence in mice. J. Virol. **67**, 4956-4963

POST, P.R., SANTOS, C.N.D., CARVALHO, R., CRUZ, A.C.R., RICE, C.M., AND GALLER, R. (1992). Heterogeneity in envelope protein sequences and N-linked glycosylation among yellow fever virus vaccine strains. *Virology* **188**, 160-167.

RANCANIELLO, V.R., BALTTIMORE, D. (1981) Science, 214:916-919

REVAILLARD, J.P., COZON, G., CZERKINSKY, C. (1992). Oral administration of immunomodulators and the mucosal immune system. Dev. Biol. Stand. 77:31-37.

RICE, C.M., GRAKOUI, A., GALLER, R., AND CHAMBERS, T.J. (1989). Transcription of infectious yellow fever RNA from full-length cDNA templates produced by in vitro ligation. T*he New Biol*. **1**, 285-296.

RUECKERT, R.R. (1990).Picornaviridae and their replication. In: 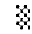 Virology 2^{nd} edn. 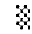 (B.N. Fields, ed.)pp. 507-548, Raven Press, New York.

SHAH, K.V. (1990). Polyomaviruses. In: 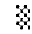 Virology 2^{nd} edn. 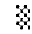 (B.N. Fields, ed.)pp. 1609-1624, Raven Press, New York.

SUMIYOSHI, H., HOKE, C., TRENT, D.W. (1992). Infectious Japanese Encephalitis Virus RNA can be synthesized from in vitro ligated cDNA template. J. Virol. 66:5425-5431.

TAGLIABUE, A., AND BORASCHI, D. (1993). Cytokines as vaccine adjuvants: Interleukin 1 and its synthetic peptide 163-171. *Vaccine* **11**, 594-595.

ULMER, J.F, DONNELLEY J.J., PARKER, S.E., RHODES, G.H, FELGNER P.L., DWARKI, V.J., GROMKOWSKI, S.H, DECK, R.R., DEWITT, C.M., FRIEDMAN, A., HAWE, L.A., LEANDER, K.R., MARTINEZ, D., PERRY, H.C., SHIVER, J.W., MONTGOMERY, D.L., LIU, M.A. (1993). Heterologous protection against influenza by injection of DNA encoding a viral protein.*SCIENCE* **259**, 1745

VENUGOPAL, K., BUCKLEY, A., REID, H.W., AND GOULD E.A. (1992). Nucleotide sequence of the envelope glycoprotein of Negishi virus shows very close homology to louping ill virus. *Virology* **190**, 515-521.

WHITBY, J.E., WHITBY, S.N., JENNINGS, A.D., STEPHENSON, J.R., AND BARRETT, A.D.T. (1993). Nucleotide sequence of the envelope protein of a Turkish isolate of tick-borne encephalitis (TBE) virus is distinct from other viruses of the TBE virus complex *J. Gen. Virol*. **74**, 921-924.

## Patentansprüche

1. cDNA enthaltend für TBEV-kodierende Sequenzen, dadurch gekennzeichnet, dass sie eine 3'-nicht-kodierende Sequenz, die mit der von Fig. 5a zu mindestens 80% homolog ist, umfasst und für ein infektiöses TBEV-RNA-Transkript kodiert.

2. cDNA nach Anspruch 1, dadurch gekennzeichnet, dass sie die 3'-nicht-kodierende Sequenz nach Fig. 5a umfaßt.

3. cDNA nach Anspruch 1 oder 2, dadurch gekennzeichnet, dass sie die für TBEV kodierenden Sequenzen abgeleitet von TBEV (W), TBEV (FE) oder Subtypen oder Varianten davon umfasst.

4. cDNA nach Anspruch 3, dadurch gekennzeichnet, dass sie die Sequenz gemäss Fig. 5 umfasst.

5. cDNA enthaltend für TBEV-kodierende Sequenzen, dadurch gekennzeichnet, dass diese die für TBEV-kodierenden Sequenzen gemäss Fig. 5 zusammen mit einer 3'-nicht-kodierenden Sequenz eines vom Stamm Neudörfl unterschiedlichen TBEV-Stammes umfasst und für ein infektiöses Transkript kodiert.

6. cDNA nach Anspruch 5, dadurch gekennzeichnet, dass diese die 3'-nicht-kodierende Sequenz des TBEV-Stammes Hypr umfasst.

7. cDNA nach einem der Ansprüche 1 bis 6, dadurch gekennzeichnet, dass diese mindestens eine die Virulenz von TBEV herabsetzende und/oder die Replikation von TBEV beeinflussende Modifikation aufweist.

8. cDNA nach Anspruch 7, dadurch gekennzeichnet, daß die Modifikation durch Substitution, Insertion oder Deletion eingeführt wird.

9. cDNA nach einem der Ansprüche 1 bis 8, dadurch gekennzeichnet, dass diese vor Position 1 ein zusätzliches G aufweist.

10. cDNA nach einem der Ansprüche 1 bis 9, dadurch gekennzeichnet, dass es eine Modifikation der Nukleinsäuresequenz an der pr(M)-Spaltstelle aufweist.

11. cDNA nach einem der Ansprüche 1 bis 9, dadurch gekennzeichnet, dass es mindestens eine Modifzierung an einer N-Glykosylierungsstelle aufweist.

12. cDNA nach einem der Ansprüche 1 bis 9, dadurch gekennzeichnet, dass es mindestens eine Modifikation gemäss Tabelle 3a aufweist.

13. cDNA nach einem der Ansprüche 1 bis 9, dadurch gekennzeichnet, dass es eine Modifikation an der Position der Aminosäure 384 des E-Proteins von TBEV aufweist.

14. cDNA nach einem der Ansprüche 1 bis 13, dadurch gekennzeichnet, dass diese zusätzlich ein für einen Immunmodulator kodierende Nukleotidsequenz umfasst.

15. cDNA nach Anspruch 14, dadurch gekennzeichnet, dass die für den Immunmodulator kodierende Sequenz ausgewählt ist aus einer für Interleukine, Diphtherietoxin, Choleratoxin oder E. coli hitzelabiles Toxin oder Pertussistoxin kodierenden Sequenz.

16. cDNA nach einem der Ansprüche 1 bis 15, dadurch gekennzeichnet, dass sie in einem rekombinanten DNA-Vektor enthalten ist.

17. cDNA nach Anspruch 16, dadurch gekennzeichnet, dass sie im rekombinanten DNA-Vektor unter Kontrolle eines Promotors ausgewählt aus den prokaryotischen Promotoren SP6, T3, T7 oder aus den eukaryotischen Promotoren von CMV, RSV, βActin, SV40 oder Adeno-2 steht.

18. Infektiöses RNA-Transkript erhältlich durch in vivo oder in vitro Transkription einer cDNA gemäß einem der Ansprüche 1 bis 17.

19. Verfahren zur Herstellung eines rekombinanten TBE-Virus, umfassend die Schritte:
- in vitro Herstellung eines RNA-Transkripts aus einer cDNA enthalten in einem rekombinanten DNA-Vektor nach Anspruch 16 und 17 und Transfektion von Wirtszellen mit dem erhaltenen RNA-Transkript und
- Gewinnen infektiöser Viruspartikel.

20. Verfahren zur Herstellung eines rekombinanten TBE-Virus, umfassend die Schritte:
- Transfektion von Wirtszellen mit einer cDNA enthalten in einem rekombinantem DNA-Vektor nach Anspruch 16 oder 17 und
- Gewinnen infektiöser Viruspartikel.

21. Rekombinantes TBE-Virus erhältlich durch ein Verfahren gemäß Anspruch 19 oder 20.

22. TBE-Impfstoff enthaltend ein rekombinantes TBE-Virus nach Anspruch 21 und einen physiologisch akzeptablen Träger.

23. Impfstoff nach Anspruch 22, dadurch gekennzeichnet, dass das TBE-Virus in attenuierter Form vorliegt.

24. Impfstoff nach Anspruch 22 oder 23, dadurch gekennzeichnet, dass das TBE-Virus in inaktivierter Form vorliegt.

25. Verwendung eines rekombinanten TBE-Virus nach Anspruch 21 als Saatvirus zur Virusvermehrung.

26. Verwendung einer Nukleinsäure nach einem der Ansprüche 1 bis 18 zur Herstellung von Impfvirus.

## Claims

1. cDNA containing sequences coding for TBEV, characterised in that it includes a 3'-non-coding sequence which is at least 80 % homologous with that of Figure 5a and codes for an infectious TBEV-RNA transcript.

2. cDNA according to claim 1, characterised in that it includes the 3'-non-coding sequence according to Figure 5a.

3. cDNA according to claim 1 or 2, characterised in that it includes the sequences coding for TBEV derived from TBEV (W), TBEV (FE) or sub-types or variants thereof.

4. cDNA according to claim 3, characterised in that it includes the sequence according to Figure 5.

5. cDNA containing sequences coding for TBEV, characterised in that this includes the sequences coding for TBEV according to Figure 5 together with a 3'-noncoding sequence of a TBEV strain different to the Neudörfl strain and codes for an infectious transcript.

6. cDNA according to claim 5, characterised in that this includes the 3'-non-coding sequence of the Hypr strain TBEV.

7. cDNA according to one of claims 1 to 6, characterised in that this has at least one modification reducing the virulence of TBEV and/or influencing the replication of TBEV.

8. cDNA according to claim 7, characterised in that the modification is introduced by substitution, insertion or deletion.

9. cDNA according to one of claims 1 to 8, characterised in that this has an additional G before position 1.

10. cDNA according to one of claims 1 to 9, characterised in that it has a modification of the nucleic acid sequence at the pr(M) cleavage position.

11. cDNA according to one of claims 1 to 9, characterised in that it has at least one modification at an N-glycosylation position.

12. cDNA according to one of claims 1 to 9, characterised in that it has at least one modification according to Table 3a.

13. cDNA according to one of claims 1 to 9, characterised in that it has a modification at the position of amino acid 384 of the E protein of TBEV.

14. cDNA according to one of claims 1 to 13, characterised in that this additionally includes a nucleotide sequence coding for an immunomodulator.

15. cDNA according to claim 14, characterised in that the sequence coding for the immunomodulator is selected from a sequence coding for interleukins, diphtheria toxin, cholera toxin or E. coli heat-sensitive toxin or pertussis toxin.

16. cDNA according to one of claims 1 to 15, characterised in that it is present in a recombinant DNA vector.

17. cDNA according to claim 16, characterised in that in the recombinant DNA vector it is under the control of a promoter selected from the prokaryotic promoters SP6, T3, T7 or from the eukaryotic promoters of CMV, RSV, βActin, SV40 or adeno-2.

18. Infectious RNA transcript which can be obtained by in vivo or in vitro transcription of a cDNA according to one of claims 1 to 17.

19. Process for producing a recombinant TBE virus comprising the steps:
- in vitro production of an RNA transcript from a cDNA present in a recombinant DNA vector according to claim 16 and 17 and transfection of host cells using the RNA transcript obtained and
- recovery of infectious virus particles.

20. Process for producing a recombinant TBE virus comprising the steps:
- transfection of host cells using a cDNA present in a recombinant DNA vector according to claim 16 or 17 and
- recovery of infectious virus particles.

21. Recombinant TBE virus which can be obtained by a process according to claim 19 or 20.

22. TBE vaccine containing a recombinant TBE virus according to claim 21 and a physiologically acceptable carrier.

23. Vaccine according to claim 22, characterised in that the TBE virus is present in attenuated form.

24. Vaccine according to claim 22 or 23, characterised in that the TBE virus is present in inactivated form.

25. Use of a recombinant TBE virus according to claim 21 as a seed virus for viral multiplication.

26. Use of a nucleic acid according to one of claims 1 to 18 to produce an inoculation virus.

## Revendications

1. ADN complémentaire contenant des séquences codant pour le virus de l'encéphalite transmise par les tiques (ETT) caractérisé en ce que celui-ci, contient une séquence 3' non codante, qui est homologue à 80 % au moins à celle de la figure 5a, et qui peut être transcrite en un ARN infectieux du virus ETT.

2. ADN complémentaire selon la revendication 1, caractérisé en ce qu'il contient la séquence 3' non codante selon la figure 5a.

3. ADN complémentaire selon la revendication 1 ou la revendication 2, caractérisé en ce que la séquence codant pour le virus ETT est dérivée du virus sauvage ETT, du virus ETT du type Extrême-Orient (FE), ou de sous-types ou de variantes de ceux-ci.

4. ADN complémentaire selon la revendication 3, caractérisé en ce qu'il contient la séquence selon la figure 5.

5. ADN complémentaire contenant des séquences codant pour le virus ETT, caractérisé en ce qu'il comporte les séquences codant pour le virus ETT selon la figure 5 ensemble avec une séquence 3' non codante d'une souche de virus ETT différente de la souche Neudörfl et en ce qu'il peut être transcrit pour donner un produit infectieux.

6. ADN complémentaire selon la revendication 5, caractérisé en ce qu'il contient la séquence 3' non codante du virus ETT de la souche Hypr.

7. ADN complémentaire selon l'une des revendications 1 à 6, caractérisé en ce qu'il comprend au moins une modification qui influe sur la virulence du virus ETT en la diminuant et/ou sur la réplication du virus ETT.

8. ADN complémentaire selon la revendication 7, caractérisé en ce que la modification est réalisée par substitution, insertion ou délétion.

9. ADN complémentaire selon l'une des revendications 1 à 8, caractérisé en ce qu'il contient un G additionnel avant la position 1.

10. ADN complémentaire selon l'une des revendications 1 à 9, caractérisé en ce qu'il présente une modification de la séquence nucléotidique au niveau du site de clivage pr(M).

11. ADN complémentaire selon l'une des revendications 1 à 9, caractérisé en ce qu'il présente au moins une modification sur un site de N-glycosylation.

12. ADN complémentaire selon l'une des revendications 1 à 9, caractérisé en ce qu'il présente au moins une modification selon le tableau 3a.

13. ADN complémentaire selon l'une des revendications 1 à 9, caractérisé en ce qu'il présente une modification à la position de l'acide aminé 384 de la protéine E du virus ETT.

14. ADN complémentaire selon l'une des revendications 1 à 13, caractérisé en ce qu'en plus, il contient une séquence nucléotidique codant pour un immunomodulateur.

15. ADN complémentaire selon la revendication 14, caractérisé en ce que la séquence codant pour un immunomodulateur est choisie parmi les séquences codant pour l'interleukine, la toxine diphtérique, la toxine du choléra ou la toxine thermolabile d'E. Coli, ou encore la toxine de la coqueluche.

16. ADN complémentaire selon l'une des revendications 1 à 15, caractérisé en ce qu'il est contenu dans un vecteur d'ADN de recombinaison.

17. ADN complémentaire selon la revendication 16, caractérisé en ce que dans le vecteur d'ADN de recombinaison, il est sous le contrôle d'un promoteur, choisi parmi les promoteurs procaryotes SP6, T3, T7 ou parmi les promoteurs eucaryotes CMV, RSV, β-actine, SV40 ou adéno-2.

18. ARN infectieux de transcription pouvant être obtenu par transcription in vivo ou in vitro d'un ADN complémentaire selon l'une quelconque des revendications 1 à 17.

19. Procédé de préparation d'un virus ETT de recombinaison, comprenant les étapes consistant à :
- produire in vitro un ARN de transcription à partir d'un ADN complémentaire présent dans un vecteur d'ADN de recombinaison, selon les revendications 16 et 17, et transfecter des cellules hôtes avec l'ARN de transcription obtenu, et
- obtenir des particules virales infectieuses.

20. Procédé de préparation d'un virus ETT de recombinaison, comprenant les étapes consistant à :
- transfecter des cellules hôtes avec un ADN complémentaire contenu dans un vecteur d'ADN de recombinaison, selon la revendication 16 ou la revendication 17 et
- obtenir des particules virales infectieuses

21. Virus ETT de recombinaison qui peut être obtenu par un des procédés selon la revendication 19 ou la revendication 20.

22. Vaccin contre l'encéphalite transmise par les tiques contenant un virus ETT de recombinaison selon la revendication 21 et un vecteur acceptable sur le plan physiologique.

23. Vaccin selon la revendication 22, caractérisé en ce que le virus ETT est présent sous une forme atténuée.

24. Procédé selon la revendication 22 ou la revendication 23, caractérisé en ce que le virus ETT est présent sous une forme inactivée.

25. Utilisation du virus ETT de recombinaison selon la revendication 21, comme inoculum de culture de virus.

26. Utilisation d'un acide nucléique selon l'une des revendications 1 à 18, pour produire un virus de vaccination.
